# EUROPEAN PATENT SPECIFICATION

(11) **EP 0 975 608 B1**
(45) Date of publication and mention of the grant of the patent: **15.06.2005**
(21) Application number: 98913417.6
(22) Date of filing: 08.04.1998
(51) Int. Cl.: C07D 243/24, A61K 31/55

(54) **HALO-ALKOXYCARBONYL PRODRUGS**
HALO-ALKOXYCARBONYLVERBINDUNGEN
PROMEDICAMENTS HALOALCOXYCARBONYLES

(30) Priority: 15.04.1997 US 843369
(43) Date of publication of application: 02.02.2000
(73) Proprietor: Genentech, Inc., South San Francisco, CA 94080-4990 (US)
(72) Inventor: BLACKBURN, Brent, San Francisco, CA 94127 (US); OLIVERO, Alan, G., Half Moon Bay, CA 94019 (US); ROBARGE, Kirk, San Francisco, CA 94117 (US)
(74) Representative: Kiddle, Simon John
(86) International application number: PCT/US1998/007092
(87) International publication number: WO 1998/046576

(56) References cited:
- WO-A-93/08174
- RUBAS W ET AL: "The effect of chemical modifications on octanol/water partition (log D) and permeabilities across Caco-2 monolayers" ADV. DRUG DELIVERY REV. (ADDREP,0169409X);97; VOL.23 (1-3); PP.157-162, 15 January 1997, GENENTECH, INC., DEPARTMENT OF PHARMACEUTICAL RESEARCH AND DEVELOPMENT, 460 POINT SAN BRUNO BOULEVARD;SOUTH SAN FRANCISCO; 94080-4990; CA; USA (US), XP002069807
- MCDOWELL ET AL: "From Peptide to Non-peptide." J.AMER.CHEM.SOC., vol. 116, no. 12, 1994, pages 5077-5083, XP002069808

## Description

### Field of the Invention

This invention relates to novel halo-alkoxycarbonyl derivatives as bioconvertable prodrug moieties for pharmaceutical agents containing a polar or basic functionality. The invention also relates to novel pharmaceutical compositions containing a prodrug comprising the novel prodrug moieties as a component, as well as to processes for producing the prodrugs. The invention further relates to methods for using the novel pharmaceutical compositions described herein.

### Description of Related Disclosures

A number of pharmaceutical agents including glycoprotein IIb/IIIa antagonists [International Publication Number 95/04057; Bondinell et al., (1994) Bioorganic & Medicinal Chem., 2(9):897-908; Cook et al., (1993) Thrombosis and Haemostasis, 70(3):531-539; Frederick et al., (1996) Circulation 93(1):129-134; Cox et al., (1996) Thrombosis and Haemostasis 75(3):503-509; Aoki et al., (1996) Throm. Res. 81(4):439-450; Carteuax et al., Throm. and Haem. 70(5):817-821; International Publication Number WO 95/14682; Hodohara et al., (1995) Pathophysiol. 2:145-151; Sall et al., (1996) Bioorganic and Medicinal Chem. Letts. 6(1):81-86; Xue et al., (1996) Bioorganic and Medicinal Chem. Letts. 6(3):339-344; Colin et al., (1994) J. Med. Chem. 37:3882-3885; Bovy et al., (1994) Bioorg. and Med. Chem. 2(9):881-895], factor Xa inhibitors [Stürzebecher et al., (1989) Thromb. Res. 54:245-252; Hara et al., (1994) Thromb. and Haem. 71(3)314-319], tissue factor-Factor VIIa, trypsin and thrombin inhibitors [U.S. Patent No. 5,599,793; Bertrand et al., (1996) 35:3147-3155; International Publication Number WO 95/35312; International Publication Number WO 96/24609; International Publication Number WO95/13274; Dickneite et al., (1995) Thromb. Res. 77(4):357-368; Mack et al., (1995) J. Enzyme Inhibition 9:73-86] possess a polar or basic functional group which is integrally responsible for the desired biological activity. For example, structurally related receptors such as integrins recognize the amino acid sequence Arg-Gly-Asp known to be expressed in the extracellular surface glycoprotein GPIIb/IIIa of platelets, endothelial cells, leukocytes, lymphocytes, monocytes and granulocytes. Pharmaceutical agents having the amino acid sequence Arg-Gly-Asp, chemically modified derivatives thereof or non-peptidal analogs, having a polar or basic functionality, compete with intracellular adhesive factors to limit or prevent a key step in the aggregatory cascade. Numerous linear or cyclic peptide derivatives containing the Arg-Gly-Asp sequence or mimics thereof have been described (see references supra; Pierschbacher and Ruoslahti,(1987) j. Biol. Chem. 262:17294; European Patent Publication No. 0406428).

In the case of many of the pharmaceutical agents this polar functional group is a nitrogen atom of, for example, a guanidine, alkyl-amidine or aryl-amidine group. This is in part due to these functionalities being good peptidomimetics for the guanidine group of arginine, an important amino acid residue found in many of the natural ligands and substrates of the indicated proteins and enzymes. Because these functionalities are highly basic (for example, pKₐ of the conjugate acid ≥ 11), they remain protonated at physiologically relevant pH's. The ionic nature of such protonated species hinders their permeability across lipophilic membranes which can reduce bioavailability when the pharmaceutical agent is administered orally.

In order to circumvent such a problem, it is often advantageous to perform a derivatization or chemical modification of the polar functionality such that the pharmaceutical agent becomes neutrally charged and more lipophilic thereby facilitating absorption of the drug. However, for the derivatization of such a polar functionality to be useful, the derivatization must be bioconvertable at the target site or sites of desired pharmacological activity and cleaved under normal physiological conditions to yield the biologically active drug. The term "prodrug" has been used to denote such a chemically modified intermediate.

The alkoxycarbonyl (carbamoyl) and acyloxymethyl carbamate groups have been investigated as prodrug moieties capable of producing a bioconvertable prodrug of amine and amidine nitrogens (for example, Saulnier, et al. (1994) Bioorg. Med. Chem, Letts. 4(16):1985-1990). Lower alkoxycarbonyl groups such as methoxycarbonyl and ethoxycarbonyl, optionally substituted phenoxycarbonyl groups, groups such as benzyloxycarbonyl and p-methoxybenzyloxycarbonyl and amide oximes have been described as prodrug moieties for these functional groups (for example, European Patent Application No. 0743320).

While the carbamoyl group has many of the desired characteristics to be utilized as a prodrug moiety, it suffers from the disadvantage that the group is not readily bioconvertable. Alkoxycarbonylamines cleave too slowly in plasma and upon systemic circulation to the respective amine to be of prlactical importance. This is partly due to the fact that mammals possess no carbamoyl specific hydrolases. Cholinesterases have been shown to slowly hydrolyze some carbamates, however the cleavage is slow and the cleaving enzyme is reversibly inhibited by the cleavage resulting in a further reduction in the rate of hydrolysis (Alexander et al., (1988) J. Med. Chem. 31:318-322).

It would be highly desirable to have a modified alkoxycarbonyl-based prodrug moiety for N-containing polar functional groups which possess all of the desirable characteristics associated with a prodrug moiety and cleave readily once in the bloodstream to liberate the free N-containing drug.

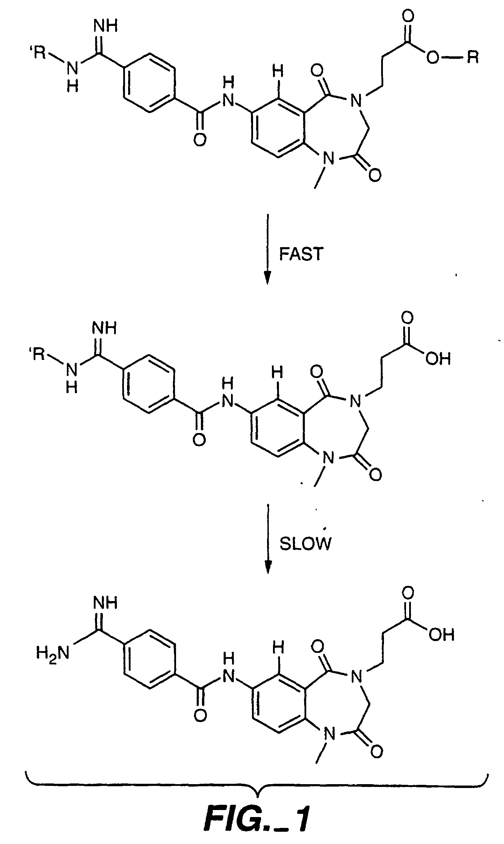

In "The effect of chemical modifications on octanal/water partition (log D) and permeabilities across Caco-2 monolayers ", Advanced Drug Delivery Reviews 23 (1997) 157-162, the authors disclose the chemical structures of seven platelet IIbIIIa antagonists (see Fig. 1).

In "From peptide to non-peptide", J. Am. Chem. Soc 1994, 116, 5077-5083, the authors disclose at page 5082, table 1, the activities of non-peptidic GPIIbIIIa antagonists, e.g. see table 1, 2g.

WO 93/08174 discloses a benzodiazopinedione derivative acting as a non-peptidyl platelet aggregation inhibitor inhibiting fibrinogen binding to the GPIIbIIIa receptor (e.g. see claim 2).

### Summary of the Invention

The present invention provides modified alkoxycarbonyl groups as bioconvertable prodrug moieties for pharmaceutical agents, drugs or medicaments having a polar or basic functional group. It is a feature of the present invention that when administered to animals including humans the pharmaceutical compositions comprising the novel prodrugs are more bioavailable following non-systemic administration than the parent pharmaceutical agents or drugs from which they are derived. Thus, the novel prodrugs which comprise the prodrug moiety of the present invention may be used wherever the parent drug or pharmaceutical agent is used and advantageously provide increased biomembrane transport and improved bioavailability from the gastrointestinal tract, rectum, skin and the eyes. The prodrugs of the invention can be incorporated into unit dosage forms such as tablets and capsules for oral delivery, as well as into suspensions, ointments and the like, depending on the particular drug or medicament and its target area.

In particular embodiments, the present invention provides such prodrug forms of pharmaceutical agents which following administration cleave in a therapeutically useful time frame and in such a manner as to enable the original parent composition to be released at its therapeutic site or sites of activity and to further permit the cleaved prodrug moiety to be metabolized in a nontoxic fashion.

According to a particular aspect of the invention the pharmaceutical composition comprising the prodrug moiety of the present invention can be described by the following formula: 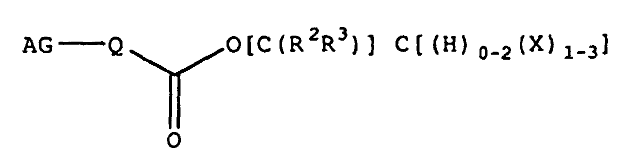 wherein [(H)₀₋₂(X)₁₋₃] is selected from the group consisting of X₃, HX₂, and H₂X and X is a halogen selected from the group consisting of F, Cl, Br, I or a combination thereof; R² and R³ are the same or different and are selected from the group consisting of H, C₁-C₄alkyl, C₁-C₄alkoxy, cyano and hato(F, Cl, Br, I)C₁-C₄alkyl and AG-Q is a biologically active pharmaceutical agent wherein Q is a basic N-containing functionality selected from the group consisting of an amino, amidino, aminoalkyleneamino, iminoalkyleneamino, and guanidino group. According to this aspect of the invention the prodrug moiety, -COO[C(R²R³)] C[(H)₀₋₂(X)₁₋₃] is linked to the N containing functionality via an N atom.

According to a preferred aspect of the present invention, halo-alkoxycarbonyl moieties, for example, 2,2,2-tri-halo-ethoxycarbonyl moities are provided as bioconvertable prodrug moieties for polar or basic functionalities in pharmaceutical agents, drugs or medicaments. Preferred polar or basic functionalities, represented by Q or B-Q herein, are N-containing functionalities having a pKₐ of the conjugate acid sufficiently high that the N-containing group is at least 10% positively charged at physiological pH. Exemplary N-containing functional groups, Q, are primary and secondary amino, amidino, aminoalkyleneamino, iminoalkyleneamino, and guanidino groups. Especially preferred are the guanidino and amidino groups and B-Q groups such as the alkyl-, alkenyl, alkynyl, substituted or unsubstituted aryl, arylalkyl, heterocyclic or heteroaromatic such as substituted or unsubstituted phenyl-, napthyl-, and pyridyl-amidino groups.

In a preferred aspect, the prodrug moiety is a 2,2,2,-trichloroethoxycarbonyl group. According to this aspect of the present invention, the prodrug moiety can be represented by the following structure: 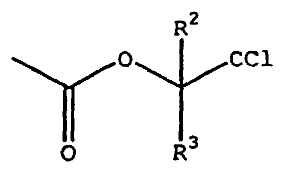 wherein the carbonyl group: 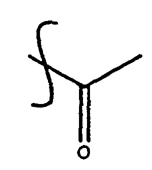 is linked to a pharmaceutical agent comprising at least one polar or basic N-containing group via the N atom.

In a preferred aspect of the present invention, the prodrug moiety can be represented by P or P' and the pharmaceutical agent can be represented by AG'-Q with Q representing an N-containing functionality of the parent agent. According to a particular embodiment, Q is an amidino group and the prodrug of the present invention is represented by the following two tautomeric structures: 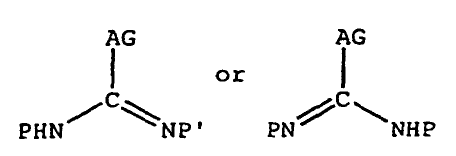 wherein AG represents a parent pharmaceutical agent substructure and wherein P and P' are the same or different and are independently selected from H or a 2,2,2,-tri-halo-ethoxycarbonyl moiety, at least one of P or P' being a 2,2,2-tri-halo-ethoxycarbonyl moiety where the ethoxycarbonyl group is linked to the N-containing functionality via the carbonyl group. According to this aspect of the invention, the ethoxy group is -OCH₂CX₃, -OCHR²CX₃, or -OCR²R³CX₃, R² and R³ being defined herein, and preferably being H.

According to a further aspect of the present invention, Q is B-Q and the pharmaceutical agent can be represented by AG-B-Q wherein B is an substituted or unsubstituted alkyl, alkenyl or alkynyl, a substituted or unsubstituted aryl, arylalkyl, heterocyclic, or heteroaromatic. In preferred embodiments B is an unsubstituted or substituted phenyl, napthyl or pyridyl. As an example of this aspect of the present invention, Q is an amidino group and the prodrug is represented by the following two tautomeric structures: 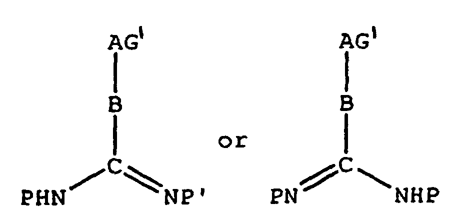 wherein AG' represents a parent pharmaceutical agent substructure and wherein P and P' are the same or different and are independently selected from H or a 2,2,2-trichloroethoxycarbonyl moiety, at least one of P or P' being a 2,2,2-trichloroethoxycarbonyl moiety where the ethoxycarbonyl group is linked to the N-containing functionality via the carbonyl group. B is preferably an unsubstituted or substituted aryl or arylalkyl such as phenyl, napthyl or pyridyl. According to this aspect of the invention, the ethoxy group is - OCH₂CCl₃, -OCHR²CCl₃, or -OCR²R³CCl₃, R² and R³ being defined herein and preferably being H.

The invention further provides novel compositions comprising the prodrugs of the present invention as well as methods of making and employing the novel prodrugs. The pharmaceutical compositions comprising the prodrugs of the present invention include all stereoisomers and pharmaceutically acceptable salts of the novel compositions.

### Brief Description of the Drawings

Figure 1: A variety of alkoxycarbonylamidines were prepared including benzyloxy- butoxy, isobutoxy, (4-pyridyl)methoxy-, 1-morpholinoethoxy-, however no increases in oral bioavailability relative to the ethoxycarbonyl compounds were observed in any parent class. As part of a program to understand why these alkoxy carbonyl prodrugs were showing low oral availability, in vitro and in vivo studies were conducted to identify whether prodrug to parent conversion was limiting the oral activity. As schematically depicted in Figure 1 when a double prodrug was incubated in rat plasma the ester functionality was cleaved by esterases almost immediately to liberate the carboxylic acid, while the ethoxycarbonyl moiety was very slow to convert to the free amidine.

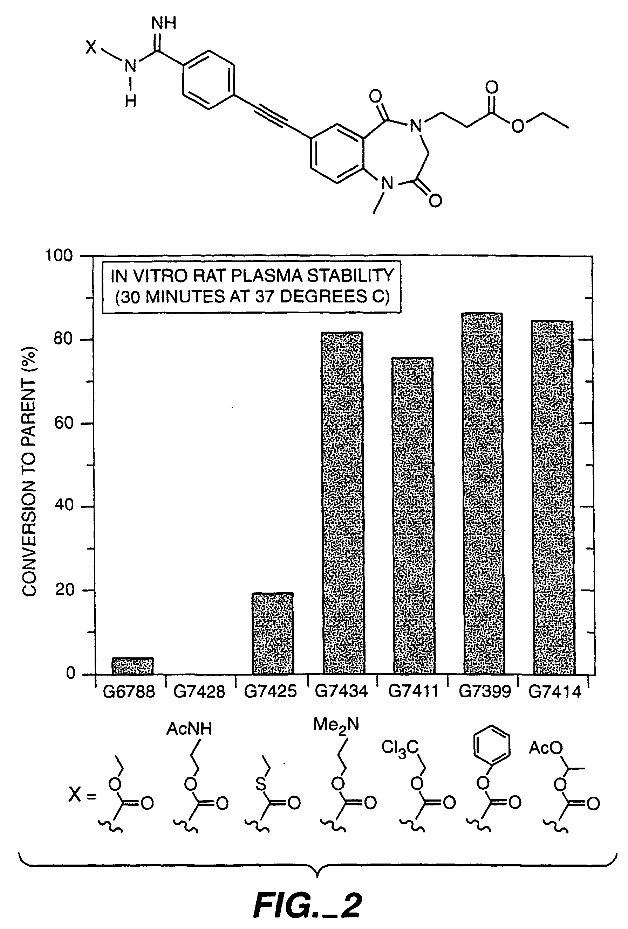

Figure 2: Figure 2 is a graphic representation of the percent conversion of various prodrugs to bioactive parent compound in rat plasma after 30 minute incubation. The ability to cleave under physiological conditions was demonstrated for various alkoxycarbonyl derivatives of GPIIb/IIIa antagonists. Unlike simple alkoxycarbonylamidines such as the ethoxycarbonyl derivative (entry 1) the 2,2,2-trichloroethoxycarbonyl modified amidines (entry 5) cleave readily in rat plasma to yield the active metabolite.

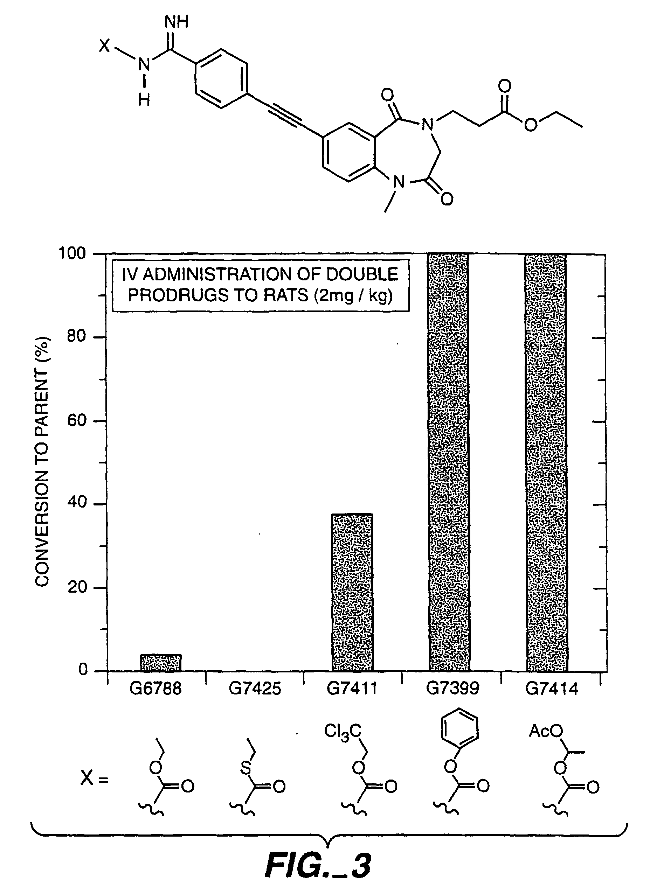

Figure 3: Figure 3 graphically depicts the bioavailability (%F) upon intravenous administration of various prodrugs in rats at 2 mg/kg. Upon delivery of alkoxycarbonylamidine prodrugs by intravenous administration, simple alkylcarbamoyl amidines do not readily convert to the active metabolite whereas the 2,2,2-trichloroethoxycarbonyl derivatives cleaves to greater than 38%.

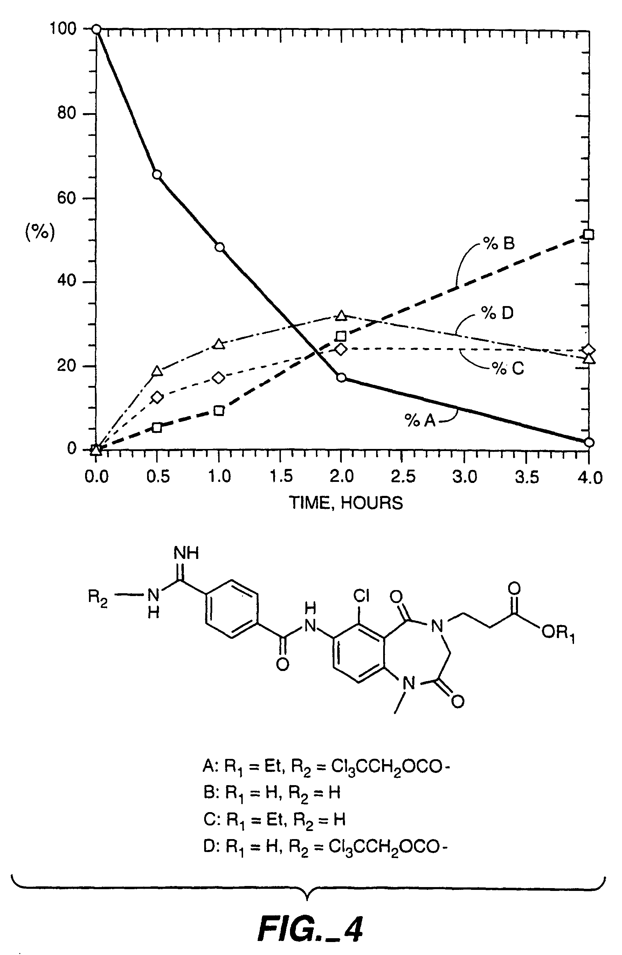

Figure 4: Figure 4 depicts graphically the conversion of various prodrugs to the active parent in dog hepatocytes at 37 C. The 2,2,2-trichloroethoxycarbonylamidines effectively cleaves in dog hepatocytes to yield the bioactive parent compound.

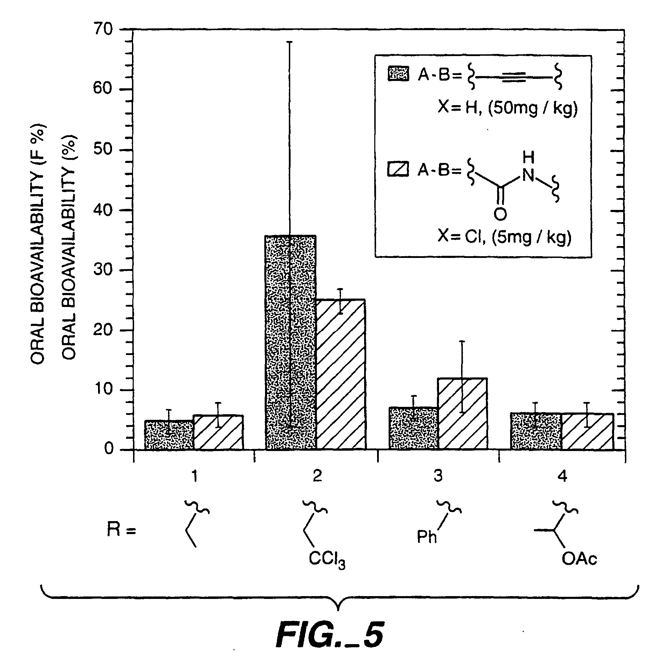

Figure 5: Figure 5 graphically depicts bioavailability (%F) of compounds of the general formula I: 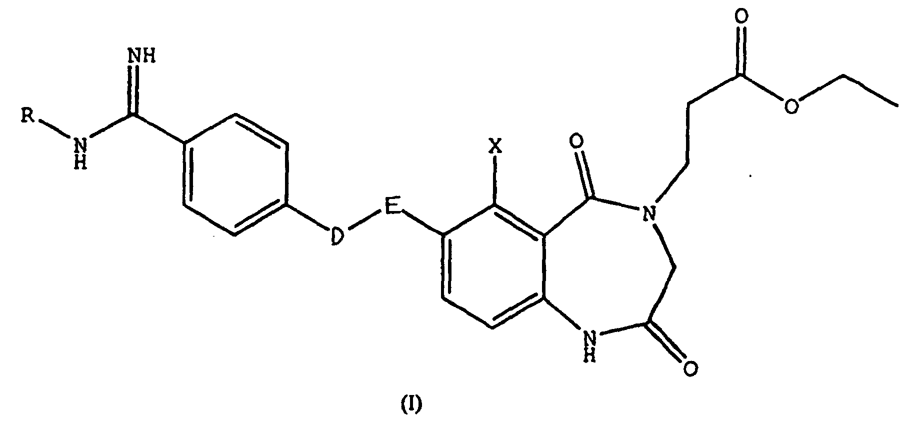 in rats after oral administration. Aryloxycarbonylamidines and acyloxy-alky-carbonylamidines are more physiologically labile than 2,2,2-trichloroethoxycarbonyl amidines (entries 6 and 7 of Figure 2 and entries 4 and 5 of Figure 3) but such chemical modifications do not significantly increase oral bioavailability in rats. The 2,2,2-trichloroethoxycarbonyl prodrug exhibits the proper balance of lability and stability.

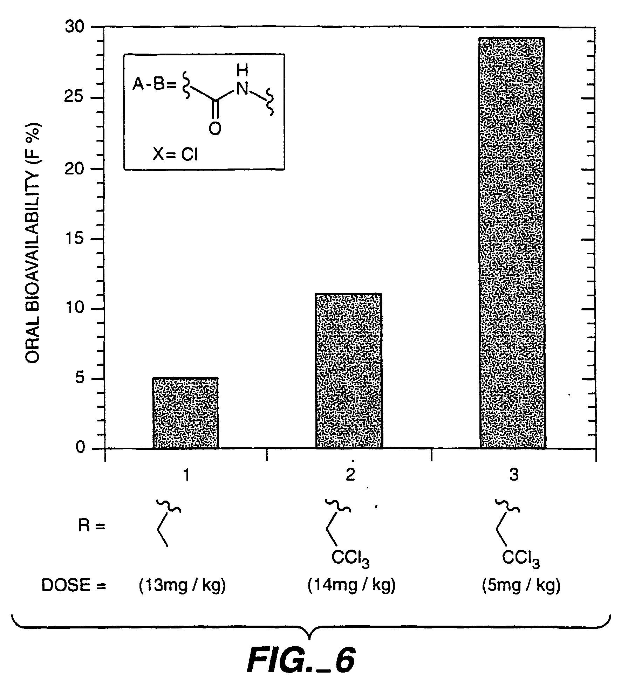

Figure 6: Figure 6 graphically depicts bioavailability of various prodrugs in dogs after oral administration. Use of the 2,2,2-trichloroethoxycarbonyl prodrug moiety doubled the oral bioavailability of compounds of the general structure (I) relative to the ethoxycarbonyl derivative when administereed orally at comparable doses to dogs. At lower doses the oral bioavailability increased to 29%.

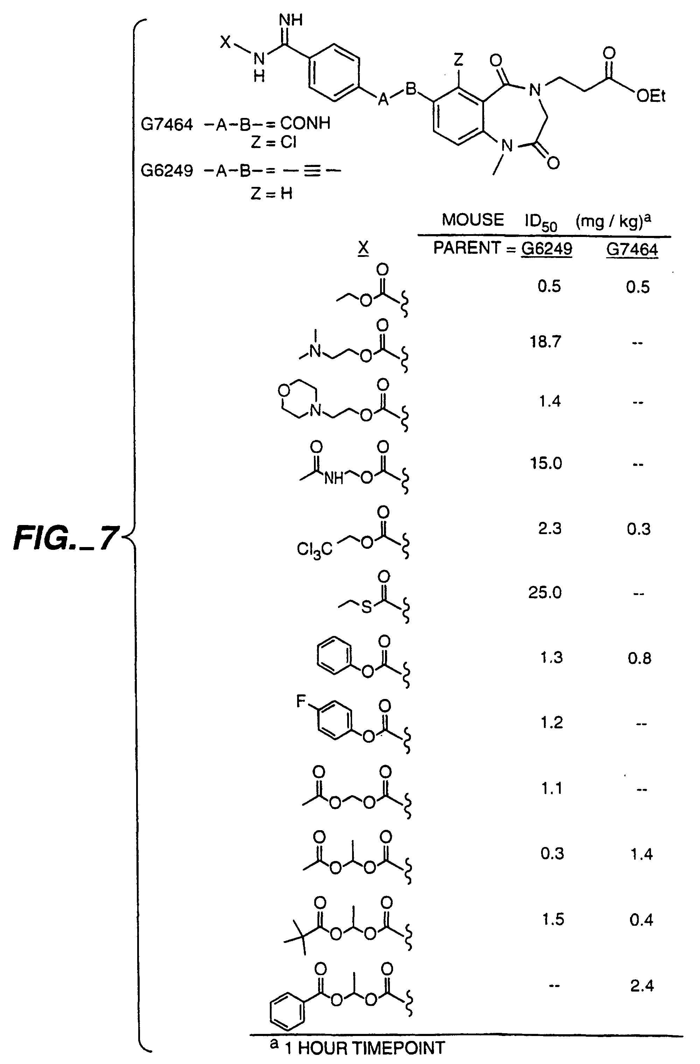

Figure 7: Figure 7 show the results of oral bioavailability assessed by dosing mice at 100 mg/kg orally and sacrificing the mice at either I or 3 hour timepoints. The mice were exsanguinated and ex vivo platelet aggregation measured by ID₅₀.

### Detailed Description of the Preferred Embodiments

### Definitions

The terms "pharmaceutical agent," "drug," "medicament" and the like are used interchangeably herein with the term "parent compound" or "parent drug" to refer to a compound, having pharmacological activity. Pharmaceutical agents or parent compounds characteristically contain at least one polar or basic nitrogen- (N-) containing functionality. It is characteristic that the pharmaceutical agent comprising at least one N-containing functionality have a pKₐ of the conjugate acid sufficiently high so that the functionalities are at least 10% positively charged at physiological pH. Functionalities that are positively charged are associated with an H ion at physiological pH. In various aspects of the present invention the pharmaceutical agent or parent drug is represented by the shorthand notation AG-Q or AG-B-Q. In this shorthand notation, AG is conveniently used in place of the parent drug "substructure" with Q, or B-Q representing the parent drug at least one polar or basic N-containing functionality as defined herein, B further defining the parent drug substructure. For example, in one embodiment, the parent drug is an amidine containing GPIIb/IIIa antagonist such as those described in International Publication No. WO 93/08174 and U.S. Patent No. 5,565,449. In this example AG would represent the parent substructure and the polar or basic N-containing functionality, e.g., an amidino group, -C(=NH)-NH₂, is conveniently described in shorthand notation by Q. As a further example, AG may contain a substituted or unsubstituted aryl or arylalkyl-amidino group represented by -B-Q.

The pharmaceutical agent is pharmaceutically active or "bioactive," by virtue of possessing a biological activity such as inhibiting platelet aggregation in the absence of the prodrug moiety of the present invention. As noted above, it is a characteristic of the pharmaceutical agent of the present invention that it contain at least one polar or basic N-containing functionality (Q) as herein defmed.

The term "basic" as used in the context of the present invention refers to a funtionality or moiety having a positive charge due to association with H ion at physiological pH.

The term "polar" as used herein refers to funtionalities or moieties not charged at physiological pH, but possess a dipole moment in an aqueous (non-lipid) environment.

"Bioavailable" as used herein means that at least some amount of the parent drug is present in the systemic circulation. Formal calculations of oral bioavailability are described in terms of an F value ("Fundamentals of Clinical Pharmacokinetics," John G. Wegner, Drug Intelligence Publications; Hamilton, Illinois 1975). F values are derived from the ratio of the concentration of the parent drug in the systemic circulation (e.g., plasma) following intravenous administration to the concentration of the parent drug in the systemic circulation after administration by a non-intravenous route (e.g., oral). Therefore, oral bioavailability within the scope of the present invention contemplates the ratio or F value of the amount of parent drug detectable in the plasma after oral administration compared to intravenous administration.

"Pharmaceutically acceptable salts" include both acid and base addition salts.

The term "prodrug" is used herein to refer to a derivative of a parent drug that has enhanced pharmaceutically desirable characteristics or properties (e.g. transport, bioavailablity, pharmacodynamics, etc.) and requires "bioconversion," i.e., cleavage of the "prodrug moiety" either spontaneously or enzymatically, within the organism to release the active parent drug.

The term "alkyl" or the prefix "alk" mean a branched or unbranched, saturated aliphatic hydrocarbon radical, having the number of carbon atoms specified, or if no number is specified, having up to about 12 carbon atoms. Examples of alkyl radicals include methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 2,2-dimethylbutyl, n-heptyl, 2-methylhexyl, and the like. The terms "lower alkyl" "C₁-C₆ alkyl" and "alkyl of I to 6 carbon atoms" are synonymous and used interchangeably. A preferred "C₁-C₆ alkyl" group is ethyl.

Substituted alkyl groups include those that are substituted by one, two or three halogen, hydroxy, amino, carboxy, acyloxy (substituents such as formyloxy, acetoxy, proprionyloxy, butyryloxy, pentanoyloxy and the like,) carbamoyl, carbamoyloxy, cyano, morpholino, or methylsulfonylamino substituents and the like.

Examples of the above substituted alkyl groups include but are not limited to, hydroxyethyl, 2-morpholinoethyl, 6-hydroxyhexyl, 2,4-dichloro(n-butyl), 2-amino(iso-propyl), 2-carbamoyloxyethyl and the like.

The term "alkenyl" means a branched or unbranched hydrocarbon radical having the number of carbon atoms designated containing one or more carbon-carbon double bonds, each double bond being independently cis, trans, or a nongeometric isomer.

The term "alkynyl" means a branched or unbranched hydrocarbon radical having the number of carbon atoms designated containing one or more carbon-carbon triple bonds.

The terms "alkoxy" or "C₁-C₁₂ alkoxy" and the like are used interchangeably herein and denote groups such as methoxy, ethoxy, n-propoxy, isopropoxy, n-butoxy, t-butoxy and like groups. Substituted alkoxy groups include those that are substituted by one, two or three halogen, hydroxy, amino, carboxy, acyloxy (substituents such as formyloxy, acetoxy, proprionyloxy, butyryloxy, pentanoyloxy and the like,) carbamoyl, carbamoyloxy, cyano, morpholino, or methylsulfonylamino substituents and the like.

The term "aryl" when used alone means a homocyclic or polycyclic aromatic radical, whether or not fused, having the number of carbon atoms designated. Preferred aryl groups include phenyl, napthyl, biphenyl, phenanthrenyl, naphthacenyl, and the like (see e.g. Lang's Handbook of Chemistry (Dean, J. A., ed) 13th ed. (1985) Table 7-2).

The term "substituted phenyl" or "substituted aryl" denotes a phenyl group or aryl group substituted with one, two or three substituents chosen from halogen(F, Cl, Br, I), hydroxy, protected hydroxy, cyano, nitro, C₁-C₆ alkyl, C₁-C₆ alkoxy, carboxy, protected carboxy, carboxymethyl, protected carboxymethyl, hydroxymethyl, protected hydroxymethyl, aminomethyl, protected aminomethyl, trifluoromethyl N-(methylsulfonylamino) or other groups specified.

Examples of the term "substituted phenyl" includes but are not limited to a mono- or di(halo)phenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono- or di(alkoxy)phenyl group, for example, 2,6-dimethoxyphenyl, 4-methoxyphenyl, 3-ethoxyphenyl, 4-(isopropoxy)phenyl, 4-(t-butoxy)phenyl, 3-ethoxy-4-methoxyphenyl and the like; 3- or 4-trifluoromethylphenyl; a mono- or dicarboxyphenyl or (protected carboxy)phenyl group such 4-carboxyphenyl; a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))-phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl and the like. Preferred substituted phenyl groups include the 2- and 3-trifluoromethylphenyl, the 4-hydroxyphenyl, the 2-aminomethylphenyl and the 3-(N-(methylsulfonylamino))phenyl groups.

The term "arylalkyl" or "aralkyl" means one, two, or three aryl groups having the number of carbon atoms designated, appended to an alkyl radical having the number of carbon atoms designated including but not limited to; benzyl, napthylmethyl, phenethyl, benzhydryl (diphenylmethyl), trityl, and the like. A preferred arylalkyl group is the benzyl group.

The term "substituted C₆-C₁₀aryl-C₁-C₈alkyl" denotes a C₁-C₈alkyl group substituted at any carbon with a C₆-C₁₀aryl group bonded to the alkyl group through any aryl ring position and substituted on the C₁-C₈alkyl portion with one, two or three groups chosen from halogen (F, Cl, Br, I), hydroxy, protected hydroxy, amino, protected amino, C₁-C₇acyloxy, nitro, carboxy, protected carboxy, carbamoyl, carbamoyloxy, cyano, C₁-C₆alkylthio, N-(methylsulfonylamino) or C₁-C₄alkoxy. Optionally the aryl group may be substituted with one, two, or three groups chosen from halogen, hydroxy, protected hydroxy, nitro, C₁-C₆alkyl, C₁-C₄alkoxy, carboxy, protected carboxy, carboxymethyl, protected carboxymethyl, hydroxymethyl, protected hydroxymethyl, aminomethyl, protected aminomethyl, or an N-(methylsulfonylamino) group. As before, when either the C₁-C₈alkyl portion or the aryl portion or both are disubstituted, the substituents can be the same or different.

Examples of the term "substituted C₆-C₁₀arylC₁-C₈alkyl" include groups such as 2-phenyl-1-chloroethyl, 2-(4-methoxyphenyl)ethyl, 2,b-dihydroxy-4-phenyl(n-hexyl), 5-cyano-3-methoxy-2-phenyl(n-pentyl), 3-(2,6-dimethylphenyl)n-propyl, 4-chloro-3-aminobenzyl, 6-(4-methoxyphenyl)-3-carboxy(n-hexyl), 5-(4-aminomethyl phenyl)-3-(aminomethyl)(n-pentyl), and the like.

The term "carboxy-protecting group" as used herein refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, t-butyl, t-amyl, trityl,4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, β-(trimethylsilyl)ethyl, β-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. Further examples of these groups are found in E. Haslam, "Protective Groups in Organic Chemistry", J. G. W. McOmie, Ed., Plenum Press, New York, N.Y., 1973, Chapter 5, and T.W. Greene, "Protective Groups in Organic Synthesis", John Wiley and Sons, New York, NY, 1981. Chapter 5. The term "protected carboxy" refers to a carboxy group substituted with one of the above carboxy-protecting groups.

As used herein the term "amide-protecting group" refers to any group typically used in the peptide art for protecting the peptide nitrogens from undesirable side reactions. Such groups include p-methoxyphenyl, 3,4-dimethoxybenzyl, benzyl, O-nitrobenzyl, di-(p-methoxyphenyl)methyl, triphenylmethyl, (p-methoxyphenyl)diphenylmethyl, diphenyl-4-pyridylmethyl, m-2-(picolyl)-N'-oxide, 5-dibenzosuberyl, trimethylsilyl, t-butyl dimethylsilyl, and the like. Further descriptions of these protecting groups can be found in "Protective Groups in Organic Synthesis", by Theodora W. Greene, 1981, John Wiley and Sons, New York.

Unless otherwise specified, the terms "heterocyclic group" or "heterocyclic" or "HET" or "heterocyclyl" are used interchangeably herein and refer to any mono-, bi-, or tricyclic saturated, unsaturated, or aromatic ring having the number of atoms designated where at least one ring is a 5-, 6- or 7-membered ring containing from one to four heteroatoms selected from nitrogen, oxygen, and sulfur. Preferably at least one heteroatom is nitrogen (Lang's Handbook of Chemistry, supra). Typically, the 5-membered ring has 0 to 2 double bonds and the 6- or 7-membered ring has 0 to 3 double bonds and the nitrogen or sulfur heteroatoms may optionally be oxidized, and any nitrogen heteroatom may optionally be quatemized. Included in the definition are any bicyclic groups where any of the above heterocyclic rings are fused to a benzene ring. Heterocyclics in which nitrogen is the heteroatom are preferred.

### Modes for Carrying out the Invention

This invention provides novel prodrugs which are mono-, di-, or tri-halo-, preferably tri-halo-alkoxycarbonyl derivatives, such as trichloroalkoxycarbonyl derivatives of pharmaceutical agents, drugs or medicaments. According to the present invention, a pharmaceutical agent, as described herein, is substituted according to, for example, the methods provided herein, to contain at least one novel prodrug moiety.

The halo-alkoxycarbonyl prodrug moieties of the present invention produce a derivative or prodrug of the parent compound which facilitates absorption by masking the ionic charge of the nitrogen containing functionality of the parent compound. Advantageously, the prodrug moiety is sufficiently stable to survive premature cleavage in the stomach, intestines, and non-target sites. The prodrug moiety, is readily cleaved at the target site (e.g. the systemic circulation for most cardiovascular-based drugs) in a biologically relevant time frame and in high efficiency.

Suitable halo-alkoxycarbonyl prodrug moieties such as the trichloroalkoxycarbonyl moieties of the present invention produce by-products after metabolic cleavage of the prodrug moiety from the parent drug that are safe, non-toxic, and easily cleared by the system. In preferred embodiments, the by products are CO₂ and an alcohol, preferably 2,2,2-trichloroethanol an agent present in drinking water as a by product of the chlorination process (Bercz et al., (1991) J. Am. Tox. 10(2):223-232).
Preferably the mechanism of prodrug cleavage does not have undesired detrimental biological repercussions (i.e. have an inhibitory effect on prodrug cleaving enzyme(s), etc.). Advantageously the prodrug moiety is easily and economically prepared.

The present invention is meant to encompass mono-, di-, and tri- halo-, and preferably, tri-halo-alkoxycarbonyl prodrug derivatives as bioconvertable prodrug moieties. By halo-alkoxycarbonyl prodrug moiety is meant a compound of general formula:

-COO[C(R²R³)] C[(H)₀₋₂(X)₁₋₃]

wherein [(H)₀₋₂(X)₁₋₃] is selected from the group consisting of X₃, HX₂, and H₂X and X is a halogen selected from the group consisting of F, Cl, Br, I or a combination thereof. Preferably the halogen is flourine or chlorine or a combination thereof and more preferably the halogen is chlorine. Preferred among the mono, di and tri-chloroalkoxycarbonyl groups are the trichloroalkoxycarbonyl groups.

Preferred among the halo-alkoxycarbonyl prodrug moieties of the present invention are straight chain or branched chain substituted or saturated aliphatic C₂ alkyoxycarbonyl groups. Preferred among the branched and straight chain alkoxycarbonyl groups are the straight chain substituted or saturated aliphatic groups. Preferred among the straight chain groups are the C₂. substituted and saturated aliphatic groups and especially the saturated and substituted 2,2,2-trichloroethoxycarbonyl prodrug moieties.

Therefore in a preferred embodiment the invention provides prodrugs moieties of the following structural formula:

-COO(CR²R³)CCl₃

The prodrug moiety -COO(CR²R³)CCl₃ 1) produces a neutrally charged prodrug derivative of the parent compound which increases permeability across lipophilic membranes, 2) can be easily and economically prepared from precursor alkyl chloroformates, 3) results in ethoxycarbonyl derivatives stable to pre-systemic cleavage and 4) the resulting by products of systemic cleavage are CO₂ and an alcohol.

In the above formula the ethoxy group is -O(R²R³C)-, wherein R² and R³ are the same or different and represent hydrogen, C₁-C₄alkoxy, C₁-C₄alkyl, cyano, and halo(F, Cl, Br, I)C₁-C₄alkyl, (ii) optionally substituted C₁-C₁₂alkyl, (iii) optionally substituted C₃-C₇alkenyl, (iv) optionally substituted C₃-C₇alkynyl, (v) optionally substituted C₃-C₁₂cycloalkyl, (vi) optionally substituted C₅-C₁₂cycloalkenyl, (vii) optionally substituted C₆-C₁₄aryl, (viii) optionally substituted C₁-C₆alkylC₆-C₁₄aryl, (ix) optionally substituted C₃-C₆alkenyl-C₆-C₁₀aryl, (x) optionally substituted heterocyclyl, (xi) optionally substituted C₁-C₆alkyl-heterocyclyl, (xii) optionally substituted C₁-C₈alkoxy, (xiii) optionally substituted C₁-C₈alkoxycarbonyl, (xiv) optionally substituted C₁-C₈thioalkoxy, (xv) optionally substituted C₃-C₁₀alkenoxy, and (xvi) optionally substituted C₆-C₁₄aryloxy, (xvii) optionally substituted C₆-C₁₄aryloxycarbonyl, (xviii) optionally substituted C₆-C₁₄arylC₁-C₆alkyloxycarbonyl, where the substituents are usually one to three R² groups.

Preferred among the 2,2,2-trichloroethoxycarbonyl prodrug moieties of the present invention are the 2,2,2-trichloroethoxycarbonyl moieties wherein the ethoxy group -OCR²R³-, R² and R³ are the same or different and represent hydrogen, C₁-C₄alkoxy, C₁-C₄alkyl, cyano, and halo(F, Cl, Br, I)C₁-C₄alkyl. Preferably R² and R³ are methyl or H or a combination thereof. Preferred among these moieties is the - HR²CO- moiety. Most preferred among the 2,2,2-trichloroethoxycarbonyl groups is -COOCH₂CCl₃.

The pharmaceutical agents are pharmaceutically active compounds comprising at least one polar or basic nitrogen-containing functionality Q. Preferred pharmaceutical agents have an N-containing functionality, Q, having a pKₐ of the conjugate acid sufficiently high so that the molecules are at least 10% positively charged (associated with an H ion) at physiological pH. Suitable parent pharmaceutical compounds comprise at least one Q group. Q may be, for example (1) a primary, or secondary, amine or imine, (2) an amidino (aminoiminomethyl) group, (3) an aminoalkyleneamino group, (4) an iminoalkyleneamino group or (5) a guanidino group. According to this aspect of the present invention the parent compound contains at least one amino group including;

-NH₂,

-NR⁴H,

where R⁴ is typically, but not limited to; (i) an optionally substituted radical selected from (a) -NR⁷R⁸, (b) - C(=NR⁹)-NR⁷R⁸, (c) -N=CR¹⁰-NR⁷R⁸, (d) -NR¹¹-CR¹⁰=NR⁹, and (e) -NR¹¹-C(=NR⁹)-NR⁷R⁸ where each R⁷, R⁸, R⁹, R¹⁰, and R¹¹ is independently selected from hydrogen, hydroxy, C₁-C₄alkoxy, C₁-C₄alkyl, cyano, and halo(F, Cl, Br, I)C₁-C₄alkyl, (ii) optionally substituted C₁-C₁₂alkyl, (iii) optionally substituted C₃-C₇alkenyl, (iv) optionally substituted C₃-C₇alkynyl, (v) optionally substituted C₃-C₁₂cycloalkyl, (vi) optionally substituted C₅-C₁₂cycloalkenyl, (vii) optionally substituted C₆-C₁₄aryl, (viii) optionally substituted C₁-C₆alkylC₆-C₁₄aryl, (ix) optionally substituted C₃-C₆alkenyl-C₆-C₁₀aryl, (x) optionally substituted heterocyclyl, (xi) optionally substituted C₁-C₆alkyl-heterocyclyl, (xii) optionally substituted C₁-C₈alkoxy, (xiii) optionally substituted C₁-C₈alkoxycarbonyl, (xiv) optionally substituted C₁-C₈thioalkoxy, (xv) optionally substituted C₃-C₁₀alkenoxy, and (xvi) optionally substituted C₆-C₁₄aryloxy, (xvii) optionally substituted C₆-C₁₄aryloxycarbonyl, (xviii) optionally substituted C₆-C₁₄arylC₁-C₆alkyloxycarbonyl, where the substituents are usually one to three R¹², each R¹² typically selected from (a) optionally substituted C₆-C₁₂aryloxy, (b) optionally substituted C₆-C₁₂arylamino, (c) optionally substituted C₆-C₁₂aroyl, (d) optionally substituted C₆-C₁₂arylthio, where the substituents are usually one to three R¹³, each R¹³ typically selected from nitro, amino, C₁-C₈alkylamino, di-(C₁-C₈)alkylamino, amidino, aminomethyleneimino, imino, imino-C₁-C₄alkyl, iminomethyleneamino, guanidino, C₆-C₁₀arylamino, C₁-C₈acylamino, C₁-C₄alkylsulfonamino, azido, cyano, hydroxy, hydroxyC₁-C₈alkyl, C₁-C₈alkoxy, phenyloxy, C₁-C₈alkanoyloxy, C₁-C₈alkanoyl, C₆-C₁₂aroyl, benzamido, phenyl, halo(F, Cl, Br, I), haloC₁-C₈-alkyl, and C₁-C₈alkyl, (e) C₁-C₈alkoxy (f) C₁-C₈alkthio (g) hato(F, Cl, Br, I), (h) hydroxy, (i) mercapto, (j) C₁-C₈alkylcarbonyl, (k) carbamoyl, (I) formyl, (m) formyloxy, (n) carboxy, (o) carb-C₁-Cgalkyloxy, (p) C₁-C₈alkanoyloxy, (q) N-(C₁-C₈)alkylcarboxamido, (r) N-(C₁-C₈),N-(C₁-C₈)dialkylcarboxamido, (s) carbamoyloxy, (t) N-(C₁-C₈)alkylcarbamoyloxy, (u) N-(C₁-C₈),N-(C₁-C₈)dialkylcarbamoyloxy, (v) C₁-C₈alkylsulfinyl, (w) C₁-C₈ alkylsulfonyl, (x) C₁-C₈alkylsulfonato, (y) sulfo, (z) sulfonamido, (aa) N-(C₁-C₈) alkylsulfonamido, (ab) N-(C₁-C₈), N-(C₁-C₈)dialkylsulfonamido, (ac) amino, (ad) C₁-C₈ alkylamino, (ae) C₁-C₈ dialkylamino, (af) C₁-C₈ acylamino, (ag) N-(C₁-C₈), N-(C₁-C₈)-diacylamino, (ah) N-(C₁-C₈)-alkyl-N-(C₁-C₈)-acylamino, (ai) formylamino, (aj) ureido, (ak) isothioureido, (al) amino-C₂-C₈ alkylthio, (am) amino-C₂-C₈alkloxy, (an) amidino, (ao) guanidino, (ap) aminomethyleneimino, (aq) imino, (ar) imino-C₁-C₄ alkyl, (as) iminomethyleneamino, (at) glycylamino, (au) glycyl, (av) phthalimido, (aw) succinimido, (ax) morpholino, (ay) C₁-C₈ alkylsulfonamido, (az) N-(C₁-C₈)-alkyl-N-(C₁-C₈)alkyl sulfonoylamino, (ba) C₁-C₈alkylsulfinamino, (bb) N-(C₁-C₈)alkyl-N-(C₁-C₈)alkylsulfinamino, (bc) C₁-C₈ lkoxyamino, (bd) C₁-C₈ alkoxyamino, (be) N-(C₁-C₈)alkyl-N-(C₁-C₈)alkoxyamino, (bf) C₃-C₇cycloalkyl, (bg) oxo, and (bh) heterocyclyl, optionally any one or two pairs of R⁴-R¹¹ may independently be joined to form one or two optionally substituted heterocyclic rings, each ring optionally fused with one or two optionally substituted homocyclic or heterocyclic rings of from four to seven atoms where any heterocyclic ring contains from one to four heteroatoms selected from N, O. and S and where any ring may be substituted with from one to three R¹³, either isolated or conjugated with other nitrogen atoms to form groups including but not limited to aminomethyleneimino;
(2) an amidino (aminoiminomethyl) group including;

   -C(=NH)-NH₂,

   -C(=NH)-NHR⁵,

   -C(-NR⁶)-NHR⁵,

   and

   -C(=NH)-NR⁵R⁶,

   where R⁵ and R⁶ are the same or different and are as defined above for R⁴,
3) an aminoalkyleneamino group including;

   -N=CH-NH₂,

   -N=CH-NHR⁵,

   and

   -N=CH-NR⁵R⁶,

   where R⁵ and R⁶, are the same or different and are the same as R⁴ defined above,
(4) an iminoalkyleneamino group, including;

   -NH-CH=NH,

   -NH-CH=NR⁵,

   and

   -NH-CR⁶=NR⁵

   where R⁵, and R⁶ are the same or different and are as defined above for R⁴,
   (5) a guanidino (aminoiminomethyleneamino) group including;

      -NH-C(=NH)-NH₂,

      -NH-C(=NH)-NR⁵H,

      -NH-C(=NH)-NR⁵R⁶,

      -NH-C(=NR⁴)-NR⁵R⁶,

      -NR⁵-C(=NH)-NR⁵R⁶,

      -NR⁵-C(=NR⁵)-NH₂,

      -NR⁵-C(=NH)-NH₂,

      -NR⁵-C(=NR⁵)-NHR⁶,

      and

      -NR⁵-C(=NH)-NHR⁶,

      where R⁵, R⁶, and R⁴ are the same or different and are as defined above for R⁴,

Preferably the parent agent contains at least one amidino (aminoiminomethyl) group including;

-C(=NH)-NH₂,

-C(=NH)-NHR⁵,

-C(=NR⁶)-NHR⁵,

-C(=NH)-NR⁵R⁶,

and

-C(=NR⁴)-NR⁵R⁶,

where R⁵, R⁶, and R⁴ are defined above, an iminoalkyleneamino group, including;

-NH-CH=NH,

-NH-CH=NR⁵,

-NH-CR⁶=NR⁵,

and

-NR⁴-CR⁶=NR⁵,

where R⁵, R⁶, and R⁴ are defined above,
and guanidino groups including;

-NH-C(=NH)-NH₂,

-NH-C(=NH)-NR⁵H,

-NH-C(=NH)-NR⁵R⁶,

-NH-C(=NR⁴)-NR⁵R⁶,

-NR⁵-C(=NH)-NR⁵R⁶,

-NR⁵-C(=NR⁵)-NH₂,

-NR⁵-C(=NH)-NH₂,

-NR⁵-C(=NR⁵)-NHR⁶,

and

-NR⁵-C(=NH)-NHR⁶,

where R⁵, R⁶, and R⁴ are defined above, and multiples thereof.

In preferred embodiments Q is B-Q such as amidino (H₂NC(=NH)-B), guanidino (H₂N-C(=NH)-NH-B), and amino, H2N-B or HNR⁴B or an imino (NH2=CHNH-B) as indicated above wherein B is a substituted or unsubstituted straight or branched chain alkyl, alkenyl or alkynyl, a substituted or unsubstituted aryl, or alkylaryl, heterocyclic, or heteroaromatic. In preferred embodiments B is an unsubstituted or substituted phenyl, napthyl or pyridyl.

Exemplary preferred Q groups include the following:
Amidino groups such as B-Q wherein Q is; 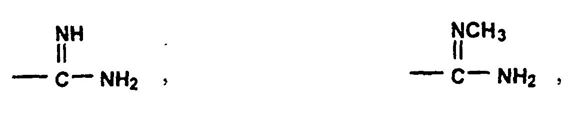 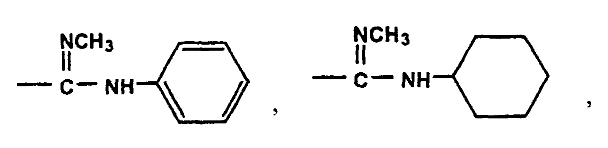 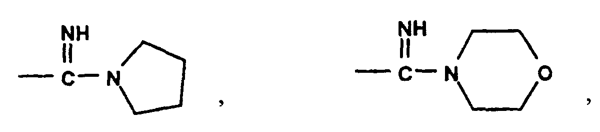 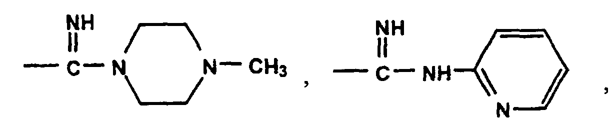
Iminoalkyleneamino groups such as B-Q wherein Q is; 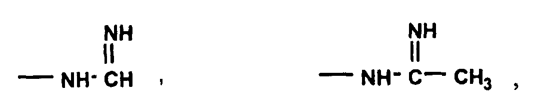 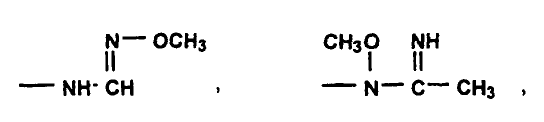 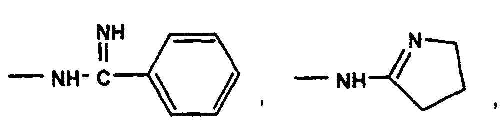 and
Guanidino groups such as B-Q; 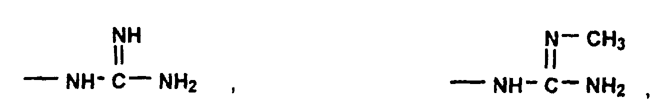 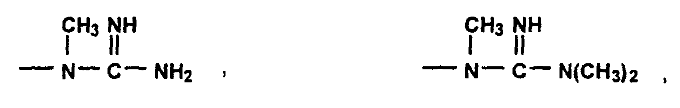 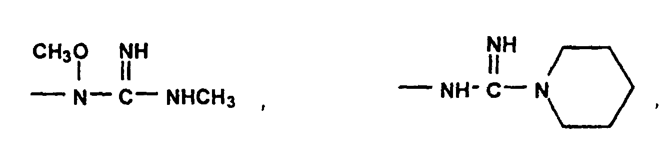 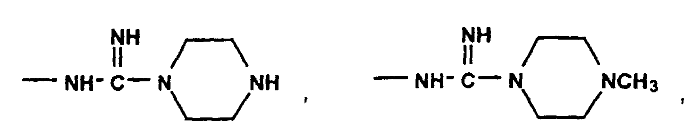 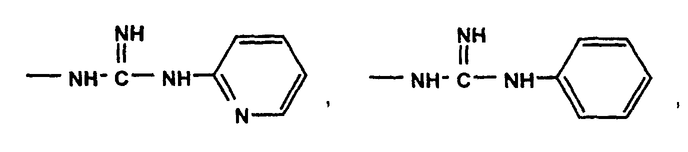 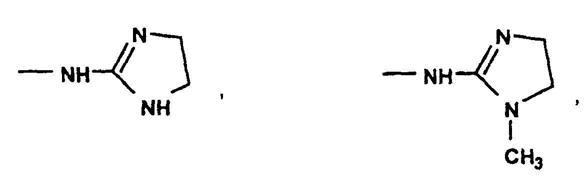 and

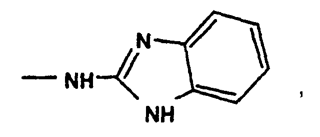

Preferred Q groups are the amidino groups and guanidino groups above and especially an B-amidino or B-guanidino wherein B is defined above. B is preferably a substituted or unsubstituted phenyl, napthyl, or pyridyl.

Listed below are various representative parent pharmaceutical agents which can be used and which contain an appropriate polar or basic function. One skilled in the art will realize that the list below is not exclusive and the invention is applicable to other equivalent drugs, pharmaceuticals, or medicaments.
Acetic acid, [[1-[2-[[4-(aminoiminomethyl)benzoyl]amino]-3-(4-hydroxyphenyl)-1-oxopropyl]-4-piperidinyl]oxy]-, (S)-
Acetic acid, [[1-[2-[[4-(aminoiminomethyl)benzoyl]amino]-3-(methyl)-1-oxopropyl]-4-piperidinyl]oxy]-, (S)-
Acetic acid, [[1-[2-[[4-(aminoiminomethyl)benzoyl]amino]-3-(methyl)-1-oxopropyl]-4-piperidinyl]oxy]-, ethyl ester (S)-
Acetamide, N-[1-[[4-(aminoiminomethyl)phenyl]methyl]-2-oxo-2-(1-piperidinyl)ethyl]-2-[(2-naphthalenylsulfonyl)amino]-, (S)-
Acetic acid, [[4-[[[6-(aminoiminomethyl)-2- quinolinyl]carbonyl]amino]cyclohexyl]oxy]-, trans-,
L-Alanine, 3-[[[3-[4-(aminoiminomethyl)phenyl]-4,5-dihydro-5-isoxazolyl]acetyl]amino]-N-(butoxycarbonyl)-, (R)-
L-Alanine, 3-[[[3-[4-(aminoiminomethyl)phenyl]-4,5-dihydro-5-isoxazolyl]acetyl]amino]-N-(butoxycarbonyl)-, (R)-, methyl ester
L-Alanine, 3-[[[3-[4-(aminoiminomethyl)phenyl]-4,5-dihydro-5-isoxazolyl]acetyl]amino]-N-[(3-methylphenyl)sulfonyl]-, methyl ester
Alanine, 3-[[[3-[4-(aminoiminomethyl)phenyl]-4,5-dihydro-5-isoxazolyl]acetyl]amino]-N-(butoxycarbonyl)-, methyl ester,
Benzamide, 4-(aminoiminomethyl)-N-[[1-[3-hydroxy-2-[(2-naphthalenylsulfonyl)amino]-1-oxopropyl]-2-pyrrolidinyl]methyl]-,[S-(R*,R*)]-,
Benzamide, 3-(aminoiminomethyl)-N-[[1-[3-hydroxy-2-[(2-naphthalenylsulfonyl)amino]-1-oxopropyl]-2-pyrrolidinyl]methyl]-,[S-(R*,R*)]-
4H-1,4-Benzodiazepine-4-propanoic acid, 7-[[4-(aminoiminomethyl)phenyl] ethynyl]-1,2,3,5-tetrahydro-1-methyl-2,5-dioxo-,
4H-1,4-Benzodiazepine-4-propanoic acid, 7-[[4-(aminoiminomethyl)phenyl] ethynyl]-1-methyl-1,2,3,5-tetrahydro-2,5-dioxo-, ethyl ester,
4H-1,4-Benzodiazepine-4-propanoic acid, 7-[[4-(aminoiminomethyl)phenyl] ethynyl]-1,2,3,5-tetrahydro-.beta.,1-dimethyl-2,5-dioxo-
4H-1,4-Benzodiazepine-4-propanoic acid, 7-[[4-(aminoiminomethyl)phenyl] ethynyl]-1,2,3,5-tetrahydro-.alpha.,1-dimethyl-2,5-dioxo-
4H-1,4-Benzodiazepine-4-propanoic acid, 7-[[4-(aminoiminomethyl)phenyl] ethynyl]-1,2,3,5-tetrahydro-.beta.,1-dimethyl-2,5-dioxo-, ethyl ester
4H-1,4-Benzodiazepine-4-propanoic acid, 7-[[4-(aminoiminomethyl)phenyl] ethynyl]-1,2,3,5-tetrahydro-.alpha.,1-dimethyl-2,5-dioxo-, ethyl ester
4H-1,4-Benzodiazepine-4-propanoic acid, 7-[[4-(aminoiminomethyl)benzoyl]amino]-1,2,3,5-tetrahydro-1-methyl-2,5-dioxo-,
4H-1,4-Benzodiazepine-4-propanoic acid, 7-[[4-(aminoiminomethyl)benzoyl]amino]-1,2,3,5-tetrahydro-1-methyl-2,5-dioxo-, ethyl ester,
4H-1,4-Benzodiazepine-4-propanoic acid, 7-[[4-(aminoiminomethyl)benzoyl]amino]-1,2,3,5-tetrahydro-.beta.,1-dimethyl-2,5-dioxo-
4H-1,4-Benzodiazepine-4-propanoic acid, 7-[[4-(aminoiminomethyl)benzoyl]amino]-1,2,3,5-tetrahydro-.beta.,1-dimethyl-2,5-dioxo-, ethyl ester
4H-1,4-Benzodiazepine-4-propanoic acid, 7-[[4-(aminoiminomethyl)benzoyl]amino]-1,2,3,5-tetrahydro-.alpha.,1-dimethyl-2,5-dioxo-
4H-1,4-Benzodiazepine-4-propanoic acid, 7-[[4-(aminoiminomethyl)benzoyl]amino]-1,2,3,5-tetrahydro-.alpha.,1-dimethyl-2,5-dioxo-, ethyl ester
4H-1,4-Benzodiazepine-4-propanoic acid, 6-chloro7-[[4-(aminoiminomethyl) benzoyl]amino]-1,2,3,5-tetrahydro-1-methyl-2,5-dioxo-,
4H-1,4-Benzodiazepine-4-propanoic acid, 6-chloro-7-[[4-(aminoiminomethyl) benzoyl]amino]-1,2,3,5-tetrahydro-1-methyl-2,5-dioxo-, ethyl ester,
4H-1,4-Benzodiazepine-4-propanoic acid, 6-chloro-7-[[4-(aminoiminomethyl) benzoyl]amino]-1,2,3,5-tetrahydro-1-methyl-2,5-dioxo-, isopropyl ester,
4H-1,4-Benzodiazepine-4-propanoic acid. 6-chloro-7-[[4-(aminoiminomethyl) benzoyl]amino]-1,2,3,5-tetrahydro-1-methyl-2,5-dioxo-, see-butyl ester,
4H-1,4-Benzodiazepine-4-propanoic acid, 6-chloro-7-[[4-(aminoiminomethyl) benzoyl]amino]-1,2,3,5-tetrahydro-.beta.,1-dimethyl-2,5-dioxo-
4H-1,4-Benzodiazepine-4-propanoic acid, 6-chloro-7-[[4-(aminoiminomethyl) benzoyl]amino]-1,2,3,5-tetrahydro-.beta.,1-dimethyl-2,5-dioxo-, ethyl ester
4H-1,4-Benzodiazepine-4-propanoic acid, 6-chloro-7-[[4-(aminoiminomethyl) benzoyl]amino]-1,2,3,5-tetrahydro-.alpha., 1-dimethyl-2,5-dioxo-
4H-1,4-Benzodiazepine-4-propanoic acid, 6-chloro-7-[[4-(aminoiminomethyl) benzoyl]amino]-1,2,3,5-tetrahydro-.alpha.,1-dimethyl-2,5-dioxo-, ethyl ester
4H-1,4-Benzodiazepine-4-propanoic acid, 6-chloro-7-[[4-(aminoiminomethyl) benzoyl]amino]-1,2,3,5-tetrahydro-.alpha.,1-dimethyl-2,5-dioxo- , isopropyl ester
4H-1,4-Benzodiazepine-4-propanoic acid, 6-chloro-7-[[4-(aminoiminomethyl) benzoyl]amino]-1,2,3,5-tetrahydro-.alpha.,1-dimethyl-2,5-dioxo- , sec-butyl ester
4H-1 ,4-Benzodiazepine-4-propanoic acid, 7-[[4-(aminoiminomethyl)phenyl] methoxy]-1,2,3,5-tetrahydro-1-methyl-2,5-dioxo-
4H-1,4-Benzodiazepine-4-propanoic acid, 7-[[4-(aminoiminomethyl)phenyl] methoxy]-1,2,3,5-tetrahydro-1-methyl-2,5-dioxo-, ethyl ester
Benzoic acid, 4-[(aminoiminomethyl)amino]-, 6-(aminoiminomethyl)-2-naphthalenyl ester,
Benzenepropanoic acid, 4-[1-[4-(aminoiminomethyl)phenyl]-2,5-dihydro-4-methoxy-2-oxo-1H-pyrrol-3-yl]-
2-Benzofuranpropanoic acid, 5-(aminoiminomethyl)-.alpha.-[4-(3-pyrrolidinyloxy)phenyl]-, ethyl ester,
Benzenecarboximidamide, 4,4'-[1,5-pentanediylbis(oxy)]bis-
Benzenecarboximidamide, 4-[2-amino-3-oxo-3-(1-piperidinyl)propyl]-, (R)-Benzoic acid, 4-methoxy-, 4-(aminoiminomethyl)phenyl ester
1H-1,4-Benzodiazepine-2-acetic acid, 7-[[[4-(aminoiminomethyl)phenyl]amino]carbonyl]-2,3,4,5-tetrahydro-3-oxo-4-(2-phenylethyl)-
1H-2-Benzazepine-4-acetic acid, 7-[[[4-(aminoiminomethyl)phenyl]methyl amino]carbonyl]-2,3,4,5-tetrahydro-3-oxo-2-(2-phenylethyl)-
1H-2-Benzazepine-4-acetic acid, 7-[[[4-(aminoiminomethyl)phenyl]methyl amino]carbonyl]-2,3,4,5-tetrahydro-3-oxo-2-(2-phenylethyl)-,methyl ester
I H-2-Benzazepine-4-acetic acid, 7-[[4-(aminoiminomethyl)benzoyl]amino]-2,3,4,5-tetrahydro-3-oxo-2-(2-phenylethyl)-, methyl ester
Benzenecarboximidamide, 4-[octahydro-3-methyl-4,6-dioxo-5-(phenylmethyl)pyrrolo[3,4-c]pyrrol-1-yl]-, (1.alpha.,3,beta.,3a.beta.,6a.beta.)-
5-Benzofurancarboximidamide, 2,2'-(1,2-ethanediyl)bis-
Carbamic acid, [[4(aminoiminomethyl)phenyl]phosphonomethyl]-, C-(phenylmethyl) ester, (R)-
Carbamic acid, [1-[[4-(aminoiminomethyl)phenyl]methyl]-2-oxo-2-(1-piperidinyl)ethyl]-, phenylmethyl ester
Cyclohexanecarboxylic acid, 4-[[[[6-(aminoiminomethyl)-2- naphthalenyl]oxy]acetyl]amino]-,
Cyclohexanecarboxylic acid, 4-[[[[6-(aminoiminomethyl)-2-naphthalenyl]oxy]acetyl]amino]-, methyl ester
Butanoic acid, 4-[[1-[[4-(aminoiminomethyl)phenyl]methyl]-2-oxo-2-(1-piperidinyl)ethyl]amino]-3-[[(4-methoxy-2,3,6-trimethylphenyl)sulfonyl] amino]-4-oxo-, [S-(R*,S*)]-
Carbamicacid,[[4(aminoiminomethyl)phenyl](diphenoxyphosphinyl) methyl]-, phenylmethyl ester, (R)-
Glycinamide, N-acetyl-4-(aminoiminomethyl)-L-phenylalanyl-L-2-cyclohexyl-N-[(1-methylpyridinium-4-yl)methyl]-, iodide
Glycinamide, N-acetyl-4-(aminoiminomethyl)-D-phenylalanyl-L-2-cyclohexyl-N-[(1-methylpyridinium-4-yl)methyl]-, iodide
Glycinamide, N-acetyl-4-(aminoiminomethyl)-L-phenylalanyl-N-[[4-(aminoiminomethyl)phenyl]methyl]-L-2-cyclohexyl-
Glycinamide. N-acetyl-4-(aminoiminomethyl)-L-phenylalanyl-L-2-cyclohexyl-N-[1-(1-methylpyridinium-4-yl)ethyl]-, iodide,
Glycine, DL-2-[4-(aminoiminomethyl)phenyl]-N-(1-D-phenylalanyl-L-prolyl)-
Glycine, N-[N-2[[[4(aminoiminomethyl)phenyl]sulfonyl]amino]-4-methyl-1-oxopentyl]glycyl]-2-phenyl-, ethyl ester
(1H-Imidazo[4,5-b]pyridine-1-carboxylic acid, 5-[[4-(aminoiminomethyl)benzoyl]amino]-2,3-dihydro-2-oxo-3-(2-phenylethyl)-, methyl ester
Morpholine, 4-[3-[4-(aminoiminomethyl)phenyl]-2-[3-[[(6,7-dimethoxy-2-naphthalenyl)sulfonyl]amino]-2-oxo-1-pyrrolidinyl]-1-oxopropyl]-
2-Naphthalenepropanoic acid, 7-(aminoiminomethyl)-.alpha.-[4-[[1-(1-iminoethyl)-3-pyrrolidinyl]oxy]phenyl]-, [S-(R*,R*)]-
2-Naphthalenepropanoic acid, 7-(aminoiminomethyl)-.alpha.-[4-[[ 1-(1-iminoethyl)-3-pyrrolidinyl]oxy]phenyl]-, [R-(S*,S*)]-
2-Naphthalenepropanoic acid, 7-(aminoiminomethyl)-.alpha.-[4-[[1-(1-iminoethyl)-3-pyrrolidinyl]oxy]phenyl]-, [R-(R*,R*)]-
2-Naphthalenepropanoic acid, 7-(aminoiminomethyl)-.alpha.-[4-(3-pyrrolidinyloxy)phenyl]-, [S-(R*,R*)]-
2-Naphthalenepropanoic acid, 7-(aminoiminomethyl)-.alpha.-[4-[[1-(1-iminoethyl)-3-pyrrolidinyl]oxy]phenyl]-, ethyl ester, including the [S-,(R*,R*)] enantiomer,
Pentanamide, 2-[[[4(aminoiminomethyl)phenyl]sulfonyl]amino]-N-[[(diphenylmethyl)amino]acetyl]-4-methyl-, (S)-
4-Pentynoic acid, 3-[[4-[[4-(aminoiminomethyl)phenyl]amino]-1,4-dioxobutyl]amino]-, ethyl ester
Phenylalanine, 3-(aminoiminomethyl)-N-[N-[(4-methylphenyl)sulfonyl]glycyl]-, methyl ester,
Phenylalaninamide, 1-[(phenylmethoxy)carbonyl]-L-prolyl-4-(aminoiminomethyl)-N-butyl-,
4-Piperidineacetic acid, 1-[[[6-(aminoiminomethyl)-2- naphthalenyl] oxy]acetyl]-
4-Piperidineacetic acid, 1-[[[6-(aminoiminomethyl)-2- naphthalenyl]oxy]acetyl]-, methyl ester
Piperidine, 1-[4-(aminoiminomethyl)-N-[N-[(4-methoxy-2,3,6-trimethylphenyl)sulfonyl]-O-(2,3,4-tri-O-acetyl-.beta.-D- ribopyranosyl)-L-seryl]-D-phenylalanyl]-
Piperidine, 1-[3-[4-(aminoiminomethyl)phenyl]-2-[3-[(2-naphthalenylsulfonyl)amino]-2-oxo-1-pyrrolidinyl]-1-oxopropyl]-, [S-(R*,R*)]-
Piperidine, 1-[3-[3-(aminoiminomethyl)phenyl]-2-[(2-naphthalenylsulfonyl)amino]-1-oxopropyl]-4-methyl-, (S)-
Piperidine, 1-[3-[3-(aminoiminomethyl)phenyl]-2-[(2-naphthalenylsulfonyl)amino]-1-oxopropyl]-4-methyl-, (R)-
1-Piperidinineacetic acid, 4-[4-[4-(aminoiminomethyl)phenyl]-1-piperazinyl]-
4-Piperidineacetic acid, 1-[[4'-(aminoiminomethyl)[1,1'-biphenyl]-4-yl]carbonyl]-, cyclohexyl ester,
4-Piperidineacetic acid, 1-[[4'-(aminoiminomethyl)[1,1'-biphenyl]-4-yl]carbonyl]-, cyclopentyl ester
1-Piperazineethanol, 4-[3-[3-(aminoiminomethyl)phenyl]-2-[(2-naphthalenylsulfonyl)amino]-1-oxopropyl]-, , (S)-
L-Phenylalanine, N-[N-[5-[4-(aminoiminomethyl)phenyl]-1-oxopentyl]-L-.alpha.-aspartyl]-
L-Phenylalanine, N-[N-[3-[[4-(aminoiminomethyl)phenoxy]methyl]benzoyl]-L-.alpha.-aspartyl]-, bis(1,1-dimethylethyl) ester
L-Prolinamide, N-methyl-D-phenylalanyl-N-[[4-(aminoiminomethyl)phenyl]methyl]-
L-Prolinamide, D-phenylalanyl-N-[[4-(aminoiminomethyl)phenyl]methyl]-
L-Prolinamide, D-phenylalanyl-N-[[4-(aminoiminomethyl)phenyl]methyl]-N-methyl-
L-Prolinamide, D-phenylalanyl-N-[2-[4-(aminoiminomethyl)phenyl]ethyl]-
L-Prolinamide, D-phenylalanyl-N-[[4-(aminoiminomethyl)phenyl]methyl]-N-methyl-
L-Prolinamide, N-[(1,1-dimethylethoxy)carbonyl]-D-phenylalanyl-N-[[4-(aminoiminomethyl)phenyl]methyl]-3-(aminoiminomethyl) phenylalanine
L-Prolinamide, N-methyl-N-[(phenylmethoxy)carbonyl]-D-phenylalanyl-N-[[4-(aminoiminomethyl)phenyl]methyl]-
L-Prolinamide, N-acetyl-4-(aminoiminomethyl)-L-phenylalanyl-L-isoleucyl-L-arginyl-L-leucyl-
L-Prolinamide, N-acetyl-L-tyrosyl-L-2-cyclohexylglycyl-4-(aminoiminomethyl)-L-phenylalanyl-L-leucyl-
L-Prolinamide, 2-[[4-(aminoiminomethyl)phenyl]methyl]-N,N-bis(1-methylethyl)-3-oxo-.beta.-alanyl-L-2-cyclohexylglycyl-L-arginyl-L-leucyl-
L-Prolinamide, N-(carboxymethyl)-D-2-cyclohexylglycyl-N-[[4-(aminoiminomethyl)phenyl]methyl]-
L-Prolinamide, 3-cyclohexyl-D-alanyl-N-[[4-(aminoiminomethyl) phenyl]methyl]-
L-Prolinamide, N-(carboxymethyl)-3-cyclohexyl-D-alanyl-N-[[4-(aminoiminomethyl)phenyl]methyl]-
2-Pyridinecarboxylic acid, 1-[3-[3-(aminoiminomethyl)phenyl]-1-oxo-2-[[(1,2,3,4-tetrahydro-2-methyl-7-isoquinolinyl)sulfonyl]amino]propyl]-1,2,3,6-tetrahydro-4-methyl-,
2-Pyridinecarboxylic acid, 1-[3-[3-(aminoiminomethyl)phenyl]-1-oxo-2-[[(1,2,3,4-tetrahydro-2-methyl-6-isoquinolinyl)sulfonyl]amino]propyl]-1,2,3,6-tetrahydro-4-methyl-, ethyl ester, [S-(R*,S*)]-
2-Pyridinecarboxylic acid, 1-[3-[3-(aminoiminomethyl)phenyl]-1-oxo-2-[[(1,2,3,4-tetrahydro-2,5-dimethyl-8-isoquinolinyl)sulfonyl]amino]propyl]-1,2,3,6-tetrahydro-4-methyl-, ethyl ester, [S-(R*,S*)]-
Octanoic acid, 8-[4-(aminoiminomethyl)phenoxy]-, ethyl ester, [S-(R*,R*)]-7-(aminoiminomethyl)-a-[4-[[1-(1-iminoethyl)-3-pyrrolidinyl]oxy]phenyl -2-naphthalenepropanoic acid
3-Oxazolidineacetic acid, 4-[[4-[[4-(aminoiminomethyl)phenyl]methoxy] phenyl]methyl]-2-oxo-, ethyl ester, (S)-
3-Oxazolidineacetic acid, 4-[[4-[[4-(aminoiminomethyl)phenyl]methoxy] phenyl]methyl]-2-oxo-, (S)-L-Tyrosinamide, N-[[4-(aminoiminomethyl)phenoxy]acetyl]glycyl-L-alpha.-aspartyl-O-methyl-,
L-Valine, N-[N-[4-[4-(aminoiminomethyl)phenoxy]-1-oxobutyl]-L-.alpha.-aspartyl]-
3-Pyrrolidineacetic acid, 5-[[[4'-[imino[(amino]methyl][1,1'-biphenyl]-4-yl]oxy]methyl]-2-oxo-, methyl ester,
1-Piperidineacetic acid, 4-[4-[4-(aminoiminomethyl)phenyl]-1-piperazinyl]-,
1,3-Piperazinediacetic acid, 4-[[[4-(aminoiminomethyl) benzoyl]amino]acetyl]-2-oxo-, 3-methyl ester.

Preferred are drugs or pharmaceuticals containing an amidino functionality used for the treatment of cardiovascular disease. More preferred still are drugs or pharmaceuticals containing amidine functionality used as antithrombotics. Most preferred are drugs or pharmaceuticals containing amidine functionality that are glycoprotein IIb/IIIa antagonists, Factor Xa, Factor VIIa, trypsin or thrombin inhibitors.

In preferred embodiments the present invention relates to prodrugs of guanidine, thioguanidine or amidine containing compounds which are pharmaceutically active and, for example, are useful in inhibiting formation of thrombin, or in inhibiting platelet aggregation, or as fibrinogen receptor antagonists, and the like, such as those described in, for example:
U.S. Patent No 5,599,793;
International Publication Number WO 95/04057;
Bondinell et al., (1994) Bioorganic & Medicinal Chem., 2(9):897-908;
Cook et al., (1993) Thrombosis and Haemostasis, 70(3):531-539;
Frederick et al., (1996) Circulation 93(1):129-134;
Cox et al., (1996) Thrombosis and Haemostasis 75(3):503-509; Aoki et al., (1996) Throm. Res. 81(4):439-450;
Carteuax et al., Throm. and Haem. 70(5):817-821; International Publication Number WO 95/14682;
Hodohara et al., (1995) Pathophysiol. 2:145-151;
Sall et al., (1996) Bioorganic and Medicinal Chem. Letts. 6(1):81-86;
Xue et al., (1996). Bioorganic and Medicinal Chem. Letts. 6(3):339-344;
Colin et al., (1994) J. Med. Chem. 37:3882-3885;
Bovy et al., (1994) Bioorg. and Med. Chem. 2(9):881-895],
Stürzebecher et al., (1989) Thromb. Res. 54:245-252;
Hara et al., (1994) Thromb. and Haem. 71(3)314-319], Bertrand et al., (1996) 35:3147-3155;
International Publication Number WO 93/08174;
International Publication Number WO 95/04057 and U.S. Patent No. 5,565,449;
International Publication Number WO 95/35312;
International Publication Number WO 96/24609;
International Publication Number WO 95/13274;
Dickneite et al., (1995) Thromb. Res. 77(4):357-368;
Mack et al., (1995) J. Enzyme Inhibition 9:73-86;
Pierschbacher and Ruoslahti, (1987) J. Biol. Chem. 262:17294; and
European Patent Application No. 89910207).

Prefered prodrugs of the present invention include 2,2,2-trichloroethoxycarbonyl derivatives of the GPIIb/IIIa antagonists described in International Publication No. WO 93/08174 and U.S. Patent 5,565,449 the disclosures of which are specifically incorporated herein by reference.

Preferred among these compounds are compounds of the general structure (I) 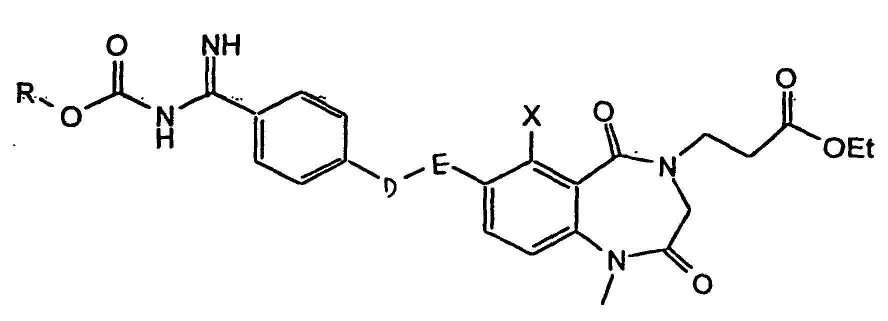 wherein D-E is: 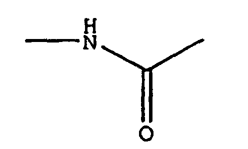 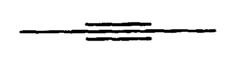 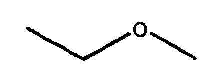 and 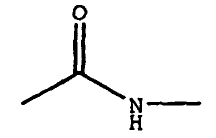 X is H and R is -CH₂CCl₃.

Whereas improvement in oral bioavailability is seen in compounds where the polar or basic N containing moiety is converted into the trichloroalkoxycarbonyl derivative as described herein, generally greater improvement in oral bioavailability is seen in compounds where both a carboxylate and an N-containing group such as an amidine group are derivatized. Therefore, the compounds of this invention are meant to include those compounds wherein both a carboxylate and at least one appropriate Q group as described herein have been derivatized.

By way of example and not limitation, carboxyl groups are commonly derivatized by a hydroxyl group, an optionally substituted alkoxy, for example a lower (Cl-6) alkylamino or a N,N-dilower (Cl-4) atklyamino, or an optionally substituted alkoxy, for a example a lower (Cl-6) alkoxy whose alkyl moiety is optional substituted by hydroxy or an optionally substituted amino, for example amino, dimethyl amino, diethylamine, diethylamino, piperidino or morphilino) halogen, a lower (Cl-6)alkoxy, a lower(Cl-6)alkythio or an optionally substituted dioxolenyl (for example 5-methyl 2 oxo 1,3-dioxolenyl or a group represented by the formula of -OCHROCOR (EP 0529 858 A 1)).

The compounds of this invention can be produced by, for example, the methods described herein below. In the following description where the parent compound contains functional groups such as carboxyl groups and other amino groups, these functional groups may, when necessary, be protected with a protective group conventionally used in the field of organic synthesis. Introduction and elimination of protecting groups may be conducted in accordance with conventional practice. The formation reaction may require the protection of the carboxy groups using one of, for example, the exter derivatives of the carboxylic acid group commonly employed to block or protect the carboxylic acid group while reactions are carried out on other functional groups. Carboxy-protecting groups are known in the art. The formation reactions may also require the protection of, for example, primary and secondary amines which may be affected by the use of a protective group such as tert-butoxycarbonyl, benxyloxycarbonyl, p-nitrobenzyloxy carbonyl and triphenylmethyl or the like.

The compounds of the present invention can be prepared by conventional methods. An exemplary method for producing the prodrugs of the instant invention can be depicted as follows:

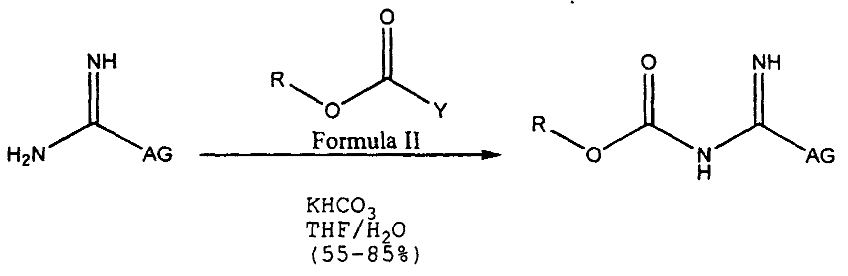

The first step of the method for preparing the prodrugs of the present invention consists of reacting a parent drug or pharmaceutical agent as described above with an alkyl formate compound of formula II where Y represents a halogen such as chlorine or an amino conjugate such as a pyrimidinium ion or a good leaving group such as a substituted phenoxy as, for example p-nitrophenoxy, dinitrophenoxy, flourophenoxy or diflourophenoxy. The reaction is carried out in solvents such as dichloromethane, dichloroethane, tetrahydrofuran, dioxane, diethyl ether, dimethoxyethane and the like or other solvents such as ethylacetate or acetonitrile. The reaction can be carried out at temperatures from -78 C to 50 C but preferably carried out at I C to room temperature. In the above exemplary schmatic R refers to the halo-alkyl group. The reaction is carried out in the presence of a base such as potassium bicarbonate, pyridine, triethylamine, 1,8-bis(dimethyl amino), napthalene, N-methylmorpholine and the like.

Compounds so obtained can be isolated from the reaction mixture by conventional separation and purification means such as extraction, concentration, neutralization, recrystalization, column chromatography and thin-layer chromatography.

The prodrugs of the instant invention can be used in a manner analogous to the parent compound. Therefore, when the AG―Q group is a thrombin inhibitor, the prodrug will be useful as a thrombin inhibitor in dosages and dosage forms as described for the particular thrombin inhibitor. If the AG―Q group is an inhibitor of platelet aggregation, the prodrug will be useful as an inhibitor of platelet aggregation in dosages and dosage forms as described for the inhibitor of platelet aggregation. It is understood that the specific dose of a compound administered according to this invention to obtain a therapeutic or prophylactic effect will, of course, be determined by the particular parent pharmaceutical agent and further by the particular circumstances surrounding the administration, including, for example, the condition being treated. Typical daily doses of the compounds are between about 0.01 mg/kg and about 100 mg/kg. The dose regime will understandably vary. For example, several above noted parent agents may be administered prophylactically as a single daily dose or in multiple doses such as between 2 and 5 times per day as appropriate.

Depending upon the AG―Q group employed the compounds of the present invention may be serine protease inhibitors and in particular may inhibit thrombin, Factor Xa, Factor VIIa and/or trypsin. Such compounds are useful for the treatment and prevention of those particular processes which involve the production and/or action of thrombin. This includes thrombotic and prothrombotic conditions in which the coagulation cascade is activated and condition wherein a large number of cells (such as neutrophls, fibroblasts, endothelial cells, smooth muscle cells) are activated. Therefore the uses include but are not limited to the treatment and prevention of peripheral arterial obstruction, acute myocardial infarction, deep vein thrombosis, pulmonary embolism, dissecting aneurysm, transient ischemic attack, restenosis, stroke and other occlusive disease or disorders such as unstable angina, disseminated intravascular coagulation, sepsis, surgical or infective shock, postoperative and post-delivery trauma, angioplasty, cardiopulmonary bypass and coronary bypass, incompatible blood transfusion, amotio placentae, thrombotic thrombocytopenic purpura, asthma, chronic or acute renal disease, diabetes, inflammations, atherosclerosis, hemolytic uremic syndrome, symmetric peripheral necrosis, and allograft rejection in mammals including human.

Such AG―Q compounds and hence the prodrugs of the instant invention can be used for enhancing the action of a thrombolytic agent and for preventing reobstruction after percutaneous transluminal coronary recanalization, preventing reobstruction after percutaneous transluminal coronary angioplasty and preventing thrombocytopenia due to dialysis, for example.

As a further example, the glycoprotein IIb/IIIa appears on the surface of platelets as a noncovalent heterodimeric complex and has been shown to interact with, fibronectin, vitronectin, von Willebrand factor, and thrombospondin. These adhesive proteins contain Arg-Gly-Asp (RGD) sequences which serve as a basic recognition feature for binding GPIIb/IIIa and certain other integrins. In various binding studies, small peptides containing the RGD fragment have been shown to successfully compete with larger proteins (see references, supra). The interaction between the particular receptor and GPIIbIIIa is the common ultimate event in the aggregation cascade regardless of the mechanism of platelet activation. Arg-Gly-Asp-based antagonists of the platelet GPIIbIIIa /receptor interaction act as a stimulus-independent inhibitor of platelet aggregation (International Publication No. WO 95/04057). Therefore the compounds and hence the prodrugs of the instant invention can be used to prevent the development of blood platelet thrombosis and can be used in the treatment and prevention of diseases and disorders involving inappropriate or non-beneficial platelet aggregation or thrombosis.

The prodrugs of the instant invention can be administered topically, orally or parenterally such as subcutaneously, intravenously, as well as by nasal, rectal or sublingual application to various mammalian species including human. In preferred embodiments the pharmaceutical composition containing the prodrug of the instant invention may be in a unit form suitable for oral use, for example, as tablets, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such composition may contain one or more agents, such as those selected from the group consisting of sweetening agents, flavoring agents, coloring agents and preserving agents, in order to provide pharmaceutically palatable preparations.

Formulations for oral use include tablets which contain the prodrug in admixture with a non-toxic pharmaceutically acceptable excipients. These excipients may be, for example, inert diluents, such as calcium carbonate, sodium chloride lactose, calcium phosphate or sodium phosphate; granulating and disintegrating agents for example maize starch, gelatin or acacia and lubricating agents for example magnesium stearate, stearic acid or talc. The tablets may be coated by known techniques to delay disintegration and absorption in the gastrointestinal tract and thereby provide a sustained action over a longer period. For example a time delay material such as glycerol monostearate or glycerly distearate may be employed. Formulation for oral use may also be presented as hard gelatin capsules wherein the prodrug is mixed with an inert solid diluent, for example calcium carbonate, calcium phosphate or kaolin, or as soft gelatin capsules wherein the active ingredient is mixed with water or an oil medium for example peanut oil, liquid paraffin or olive oil.

Aqueous suspensions usually contain the prodrug in admixture with appropriate excipients. Such excipients are suspending agents, for example sodium carboxymethycellulose, methylcellulose, hydroxypropylmethylcellulose, sodium alginate, polyvinylpyrrolidone, gum tragacanth and gum acacia, dispersing or wetting agents which may be a naturally-occurring phosphatide, for example, lecithin; a condensation product of an alkylene oxide with a fatty acid, for example, polyoxyethylene stearate, a condensation product of ethylene oxide with a long chain aliphatic alcohol for example heptadecaethyleneoxycetanol; a condensation product of ethylene oxide with a partial ester derived from fatty acids and a hexitol such as polyoxyethlene sorbitol monooleate or a condensation product of ethylene oxide with a partial ester derived from fatty acids and hexitol anhydrides, for example, polyoxyethylene sorbitol monooleate. The aqueous suspension may also contain one or more preservatives, for example, ethyl, n-propyl, or a p-hydroxybenzoate; one or more coloring agents; one or more flavoring agents; and one or more sweetening agents such as sucrose or saccharin.

Oily suspensions may be formulated by suspending the prodrug in a vegetable oil, for example, arachis oil, olive oil, sesame oil, peanut oil or coconut oil, or in mineral oil such as liquid paraffin. The oily suspensions may contain a thickening agent, for example beeswax, hard paraffin or cetyl alcohol. Sweetening agents such as those set forth above and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent suspending agent and one or more preservatives. Suitable dispersing or wetting agents and suspending agents are exemplified above. Additional excipients may also be present.

The prodrugs may also be administered in the form of suppositories. These compositions can be prepared by mixing the prodrug with a suitable non-irritating excipient which is solid at ordinary temperatures but liquid at the rectal temperature and will therefore melt in the rectum to release the drug, for example cocoa butter and polyethylene glycols.

The following examples are offered by way of illustration and not by way of limitation.

### EXAMPLES

### Example 1

### Introduction

A GPIIb/IIIa binding epitope on fibrinogen consisting of the sequence Arg-Gly-Asp (RGD) has been identified and a number of synthetic peptides containing this sequence have been shown to be GPIIb/IIIa antagonists (see, Detailed Description of the Preferred Embodiments). Based upon the structural features of this RGD epitope, potent nonpeptidal antagonists of the general structure I have been prepared.

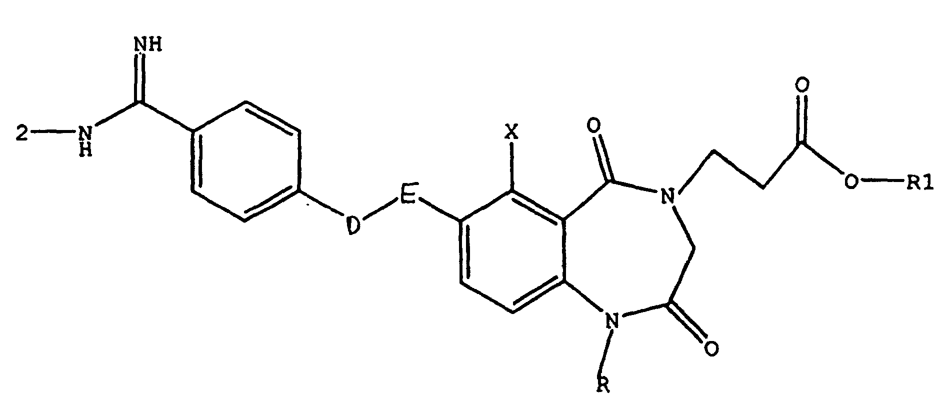

Structure I
where, for example:

| | | | |
|---|---|---|---|
| G6191 | D-E=YX=H | R=Me, R1=R2=H | IC₅₀(PA) = 59nM |
| G6249 | D-E=W | X=H R=Me, R1=R2=H | IC₅₀(PA) = 120nM |
| G6703 | D-E=XX=H | R=Et, R1=R2=H | IC₅₀(PA) = 76nM |
| G7464 | D-E=YX=Cl | R=Me, R1=R2=H | IC₅₀(PA) = 85 nM |

Where V, W, X and Y are 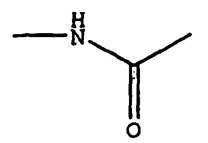 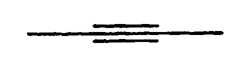 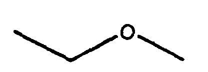 and 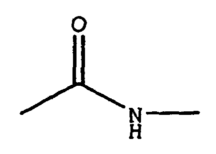 , respectively and X is H for VWX and X is Cl for Y.

IC₅₀ are measured as ADP induced aggregation in citrated plasma.

### EXAMPLE 2

The preparation of mono and and double prodrugs of these materials which masked the ionic charges were prepared and tested for oral bioavailability.

### Methods

Mouse ID₅₀ were calculated from mice dosed at 100 mg/kg orally and sacrificed at either I or 3 hours timepoints, exsanguinated and ex vivo platelet aggregation measured to determine ID₅₀.

Compounds which had a mouse ID₅₀ less than 2 mg/kg were dosed in rats (50 mg/kg) to determine their oral bioavailability (%F).

### Results

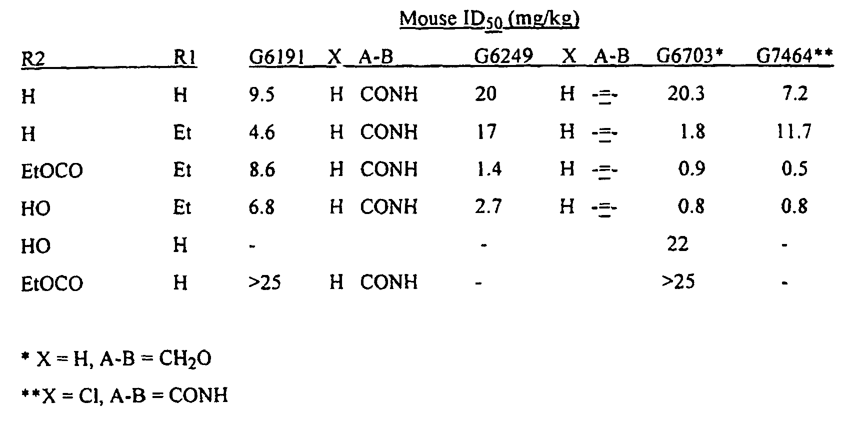

| Rat Oral Bioavailability (F) | | | | | | |
|---|---|---|---|---|---|---|
| R2 | R1 | Parent= | G6191 | G6249 | G6703 | G7464 |
| H | H | | <0.5% | - | - | - |
| H | Et | | - | - | 2% | 2% |
| EtOCO | Et | | 1% | 5% | 6% | 6% |
| HO | Et | | - | - | 14% | 7% |

Because of this slow conversion less that 5% of the active drug was produced under these conditions. This result was confirmed in vivo by injecting rats with the double prodrug and measuring the amount of active metabolite produced. Again it was observed that little of the ethoxycarbonyl moiety was hydrolyzed to the amidine consistent with in vitro plasma stability results. ,

### EXAMPLE 3

### Introduction

The compounds of Examples 4 through 9 were prepared according to the following procedure

### Methods

**General Procedure for Preparing N-2,2,2-Trichloroethoxycarbonyl Amidines.** The amidino ester (1 mmole) and potassium bicarbonate (5 mmole) are dissolved in a mixture of tetrahydrofuran (6 ml) and water (2 ml). 2,2,2-trichloroethyl chloroformate (1.2 mmole) is added dropwise with vigorous stirring. The reaction is allowed to stir for one hour, poured into a separatory funnel and extracted three times with ethyl acetate (50 ml). The combined organic layer is washed once with saturated sodium bicarbonate solution, dried over anhydrous sodium sulfate, filtered and the ethyl acetate removed *in vacuo.* The crude product is purified using flash chromatography (ethyl acetate) to yield the final product as a white solid.

### Example 4

### 4H-1,4-Benzodiazepine-4-propanoic acid, 7-[[4-(N-2,2,2-trichloroethoxycarbonylaminoiminomethyl)phenyl]methoxy]-1,2,3,5-tetrahydro-1-methyl-1-2,5-dioxo-, ethyl ester;

Yield = 74% , m.p.: 82 °C, ¹HNMR (CDCl₃): 9.5 2(1H, bs, NH), 7.93 (2H, d, J= 8.3, ArH), 7.5 (2H, d, J= 8.3 Hz, ArH), 7.37 (1H, d, J= 2.9 Hz, ArH), 7.20 (1H, d, J= 8.8 Hz, ArH), 7.12 (1H, dd, J= 8.8, J= 3 Hz, ArH), 5.08 (2H, s, CCl3), 4.83 (2H, s, PhCH₂O), 4.18, (2H, q, J=7.3 Hz, CH₃CH₂O), 3.6-4.05 (6H, complex multiplet, CH₂N), 2.72 (2H, dd, J= 6.8 Hz, J= 5.9 Hz, CH2CO), 1.25 (3H, t, J= 6.8 Hz, CH₃), 1.17 (3H, t, J= 7.3 Hz, CH₃). ; ¹³CNMR (CDCl₃): 171.20, 168.80, 167.83, 166.70, 162.99, 155.76, 144.10, 133.83, 133.43, 130.74, 127.84, 127.34, 123.25, 120.05, 114.61, 75.25, 69.47, 60.76, 60.37, 52.52, 45.08, 42.70,, 32.67, 14.08, 13.29.; MS (FAB+): 627 (100, M+H), 628, (35), 629 (100), 629 (30), 631 (33).
Exact Mass: Calculated for C₂₇H₃₀N4O₇Cl₃: 627.1180; Found: 627.1191

### Example 5

### 4H-1,4-Benzodiazepine-4-propanoic acid, 6-chloro-7-[[4-(N-2,2,2-trichioroethoxycarbonylaminoiminomethyl)benzoyl]amino]-1,2,3,5-tetrahydro-.beta.,1-dimethyl-2,5-dioxo-, ethyl ester;

Yield = 79%; ¹H-NMR (CDCl₃): 9.50 (1H, bs, OCONH), 8.62 (1H, d, J= 9.3 Hz, ArH), 8.60 (1H, s, CONH), 8.05 (2H, d, J= 8.3 Hz, ArH), 7.94 (2H, d, J= 8.3H, ArH), 7.22 (1H, d, J= 9.3 Hz), 4.87 (2H, s, Cl₃CCH₂O), 4.14 (2H, q, J= 7.0 Hz, CO₂CH₂), 4.08 (1H, d, J=15.1 Hz, COCH₂N), 4.03 (1H, dd, J= 7.8, J= 13.9 Hz, NCH₂CH₂CO), 3.86 (1H, d, J= 15.1 Hz, COCH₂N), 3.85 (1H, m. NCH₂CH₂CO), 3.38 (3H, s, NCH₃), 2.73 (2H, m, CH₂CO₂Et), 1.27 (3H, t, J= 7.1 Hz, OCH₂CH₃).; MS (FAB+): 660 (70, M+H), 661 (30), 662 (100), 663 (30), 664 (50);
Exact Mass: Calculated for C₂₆H₂₆Cl₄N₅O₇: 660.0507; Found: 660.0597;
Combustion Analysis : Calculated forC₂₆N₂₅Cl₄N₅O₇: C, 47.22, H, 3.81, N 10.59; Found:C, 47.56, H, 4.11, N, 10.27

### Example 6

### 4H-1,4-Benzodiazepine-4-propanoic acid, 7-[[4-(N-2,2,2-trichloroethoxycarbonylaminoiminomethyl)phenyl]ethynyl]-1-methyl-1,2,3,5-tetrahydro-2,5-dioxo-, ethyl ester,

Yield = 58%; m.p.: 102 °C; ¹H-NMR (CDCl₃): 9.50 (1H, bs, OCONH), 8.03 (1H, d, J= 2.0 Hz, ArH), 7.93 (2H, d, J= 8.5 Hz, ArH), 7.65 (1H, dd, J= 2.0 Hz, 8.3 Hz, ArH), 7.60 (2H, d, J= 8.4, ArH), 7.19 (1H, d, J= 8.5 Hz. ArH), 4.87 (2H, s, Cl₃CCH₂O), 4.16 (2H, q, J= 7.1 Hz, CO₂CH₂), 4.05 (1H, d, J= 15.4 Hz, COCH₂N), 3.94 (3H, m, COCH₂N, NCH₂CH₂), 3.40 (3H, s, NCH3), 2.72 (2H, m, CH₂CO₂Et), 1.27 (3H, t, J= 7.1Hz, CO₂CH₂CH₃); ¹³C-NMR (CDCl₃): 171.8, 168.8. 166.3. 163.0, 141.0, 134.89. 134.23, 133.9, 131.78, 128.9, 127.58, 127.5, 121.11, 120.5, 95.5, 90.5, 75.24, 60.88, 52.0, 45.0, 35, 32.74. 14.13.; MS (FAB+): 607 (100 M+H), 608 (50), 609 (100), 610 (40), 611 (40)
Exact Mass: Calculated for C₂₇H₂₆Cl₃N₄O₆: 607.0839; Found: 607.0885;
Combustion Analysis: Calculated for C₂₇H₂₅Cl₃N₄O₆:C, 53.35, H, 4.15, N, 9.22 Found: C, 52.71, H, 4.24, N, 8.99.

### Example 7

### 4H-1,4-Benzodiazepine-4-propanoic acid, 6-chloro-7-[[4-(N-2,2,2-trichloroethoxycarbonylaminoiminomethyl)benzoyl]amino]-1,2,3,5-tetrahydro-1-methyl-2,5-dioxo-, sec-butyl ester,

Yield: 50%; ¹H-NMR (CDCl₃): 9.50 (1H, bs, OCONH), 8.65 (1H, d, J= 9.3 Hz, ArH), 8.57 (1H, s, CONH), 8.03 (2H, d, J = 8.3 Hz, ArH), 7.98 (2H, d, J= 8.3H, ArH), 7.19 (1H, d, J= 9.3 Hz), 4.85 (2H, s, Cl₃CCH₂O), 4.08 (3H, m,), 3.86 (4H, m,), 3.36 (3H, s, NCH₃), 2.75 (2H, m, CH₂CO₂Et), 1.52 (2H, m), 1.19 (3H, m , CH₃), 0.87 (3H, m, CH₃).; MS (FAB+): 688 (33, M+H), 689 (17), 690 (40), 691 (17), 692 (20),

### Example 8

### 4H-1,4-Benzodiazepine-4-propanoic acid, 6-chloro-7-[[4-(N-2,2,2-trichloroethoxycarbonylaminoiminomethyl)benzoyl]amino]-1,2,3,5-tetrahydro-.alpha.,1-dimethyl-2,5-dioxo-, ethyl ester:

Yield :70%; ¹H-NMR (CDCl₃): 9.50 (1H, bs, OCONH), 8.62 (1H, m, ArH), 8.60 (1H, s, CONH), 8.05 (2H, m, , ArH), 7.94 (2H, m, ArH), 7.22 (1H, t, J= 9.3 Hz), 7.00 (1H, bs); 4.87 (2H, s, Cl₃CCH₂O), 4.1-4.3 (4H,m; CO₂CH₂, COCH₂N, NCH₂CH₂CO), 3.4-4.0 (2H, m, COCH₂N), 3.38 (3H, s, NCH₃), 2.93 (1H, m, CHCO₂Et), 1.27 (6H, m, 2 x CH₃). MS (FAB+): 674 (m+H), 675,676,

### Example 9

### 4H-1,4-Benzodiazepine-4-propanoic acid, 6-chloro-7-[[4-(aminoiminomethyl) benzoyl]amino]-1,2,3,5-tetrahydro-1-methyl-2,5-dioxo-, isopropyl ester,

Yield: 45%; ¹H-NMR (CDCl₃): 9.50 (1H, bs, OCONH), 8.67 (1H, d, J= 8.9 Hz, ArH), 8.61 (1H, s, CONH), 8.10 (2H, d, J= 9.5 Hz, ArH), 8.01 (2H, d, J= 9.5H, ArH), 7.22 (1H, d, J= 8.9 Hz), 5.02 (1H, m, OCH(CH₃)₂), 4.88 (2H, s, Cl₃CCH₂O), 4.10 (1H, d, J=14.1 Hz, COCH₂N), 4.02 (1H, dd, J= 7.8, J= 13.9 Hz, NCH₂CH₂CO), 3.86 (1H, d, J= 14.1 Hz, COCH₂N), 3.85 (1H, m, NCH₂CH₂CO), 3.39 (3H, s, NCH₃), 2.73 (2H, m, CH₂CO₂Et), 1.24 (6H, q, J= 6.3 Hz, OCH(CH₃)₂).; MS (FAB+): 674 (m+H), 675, 676,

### Example 10

### In Vitro Plasma Stability

### Introduction

In order to test the whether the above modified alkoxycarbonyl amidines possessed increased physiological lability, a number of compounds were submitted to in vitro plasma stability studies.

### Methods

Compounds were incubated in rat plasma at 37 C for 30 minutes. The samples were then analyzed by HPLC to determine the amount of parent produced.

### Results

The results for the G6249 based double prodrugs are shown in Figure 2.

### Conclusions

The electronically activated alkoxycarbonyl amidines (e.g trichloroethoxy, phenoxy) and the acyloxyalkoxy derivatives showed very good conversion (>10X) in comparison to the ethoxycarbonyl derivative. In the case of the acetoxyethoxy derivative almost complete conversion to the parent was observed within 30 minutes. Although the thioethoxycarbonyl derivative was superior to the simple ethoxycarbonyl derivative, this prodrug was poorer than most of the other derivatives. Similar plasma stability experiments with human plasma were consistent with the above results but absolute values were smaller, due in part to the lower levels of hydrolytic enzymes present in human plasma. Unlike simple alkoxycarbonylamidines such as ethoxycarbonyl derivative (Figure 2, entry 1), the 2,2,2-trichloroethoxycarbonyl modified amidines (figure 2, entry 5) cleave readily in rat plasma to yield the active metabolite (figure 2). Similar enhancement in extent of cleavage was noted in human plasma with the 2,2,2-trichloroethoxy derivative cleaving to the active metabolite (3%) in 30 minutes where no active metabolite was observed with incubation of the ethoxycarbonyl derivative.

Similar in vitro results show that the 2,2,2-trichloroethoxycarbonylamidine effectively cleaves in dog hepatocytes to yiel the active metabolite (Figure 4).

### Example 11

### In Vivo Stability

### Introduction

Studies were undertaken to assess the lability of the prodrugs in vivo.

### Methods

To assess the lability of these modified alkoxycarbonyl prodrugs, several of the double prodrugs were injected at 2 mg/kg i.v. into rats and the percent conversion to the parent compound in the isolated plasma calculated after 6 hours.

### Results

The results are presented in Figure 3.

### Conclusion

Consistent with the in vitro results, the acyloxyethoxy derivative showed quantitative conversion to the parent within 6 hours. Similarly the phenoxycarbonyl derivative was quantitatively converted to the parent. The trichloro ethoxy derivative was greater than 8 times more labile than the ethoxy derivatve.

### Example 12

### Oral Bioavailability

### Introduction

The double prodrugs where the amidine functionality is masked as a physiologically labile alkoxycarbonyl amidine were screened in a mouse assay to determine their oral bioavailability.

### Methods

Oral bioavailability was assessed by dosing mice at 100 mg/kg orally and sacrificing the mice at either 1 or 3 hour timepoints. The mice were exsanguinated and ex vivo platelet aggregation measured by ID₅₀.

### Results

Figure 7 summarizes the ID₅₀ results.

### Conclusion

From the data shown in Figure 7 it can be seen that the acetoxyethoxycarbonyl derivative had a better ID₅₀ than the other derivatives in the G6249 series. The trichloroethoxycarbonyl and the trimethyl acetoxyethoxycarbonyl prodrugs had a positive effect on the G7464 series.

### Example 13

### Oral Bioavailability

### 5

### Methods

Oral bioavailability was assessed by dosing dogs or rats at various concentrations orally and assessing the oral bioavailability at either 1 or 3 hour timepoints.

### Results

The results are presented in Figure 5 and Figure 6.

### Conclusion

Of the various prodrugs prepared, the trichloroethoxy derivatives had the most profound effect on improving the oral bioavailability in the rats. While the acyloxyalkoxycarbonyl and the phenoxy carbonyl compounds had shown good conversion to the corresponding parent in plasma and i.v. prodrug experiments, they did not show good oral bioavailability in rats. It could be that these prodrugs are too labile and are prematurely hydrolyzed in the stomach or intestines prior to transport. It was only the trichloroethoxycarbonyl prodrugs that showed enhanced oral bioavailability in the rat. The trichloroethoxycarbonyl prodrugs provided a dramatic increase in oral bioavailability relative to their ethoxycarbonyl analogs.

Figure 6 graphically depicts bioavailability of various prodrugs in dogs after oral administration. Use of the 2,2,2-trichloroethoxycarbonyl prodrug moiety doubled the oral bioavailability of compounds of the general structure (1) relative to the ethoxycarbonyl derivative when administereed orally at comparable doses to dogs. At lower doses the oral bioavailability increased to 29%.

## Claims

1. A prodrug compound of the formula: 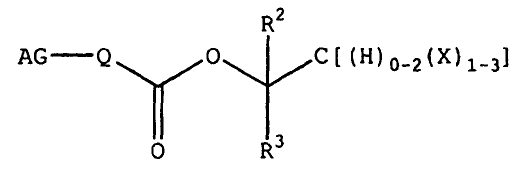 wherein [(H)₀₋₂(X)₁₋₃] is selected from the group consisting of X₃, HX₂, and H₂X and X is a halogen selected from the group consisting of F, Cl, Br, I or a combination thereof; R² and R³ are the same or different and are selected from the group consisting of H, C₁-C₄alkyl, C₁-C₄alkoxy, cyano, halo(F, Cl, Br, I)C₁-C₄alkyl and aryl, AG-Q is a pharmaceutical agent wherein Q is a basic N-containing functionality selected from the group consisting of an amino, amidino, aminoalkyleneamino, iminoalkyleneamino, and guanidino group wherein the prodrug moiety is linked to the N-containing functionality via a N atom.

2. The compound of claim 1 wherein X is Cl and R² and R³ are the same or different and are selected from the group consisting of H, C₁-C₄alkyl and aryl.

3. The compound of claim 2 wherein the C₁-C₄alkyl is selected from the group consisting of methyl, ethyl, propyl, and butyl.

4. The compound of claim 3 wherein the C₁-C₄alkyl is methyl.

5. The compound of claim 4 wherein R² and R³ are the same.

6. The compound of claim 5 wherein R² and R³ are H.

7. The compound of claim 6 where [(H)₀₋₂(X)₁₋₃] is X₃.

8. The compound of claim 1 wherein Q is selected from the group consisting of an amidino and a guanidino group.

9. The compound of claim 8 wherein Q is an amidino group for linking to the prodrug moiety and is selected from the group consisting of:

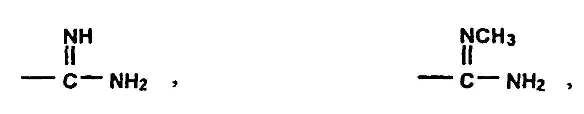

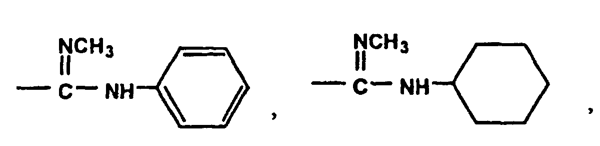

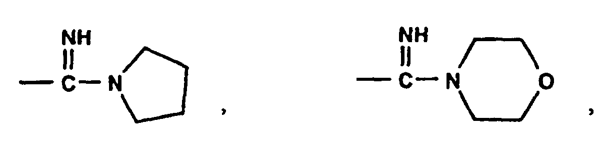

10. The compound of claim 8 wherein Q is selected from the group consisting of:

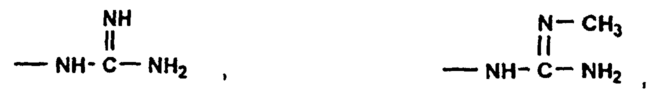

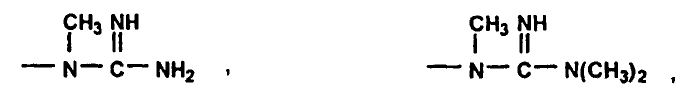

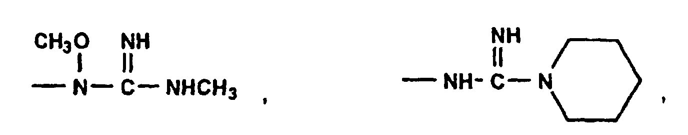

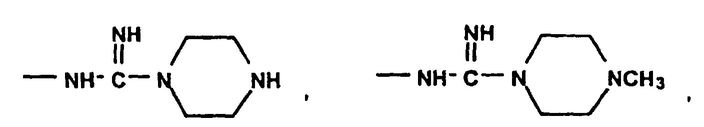

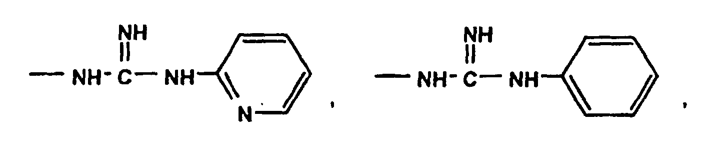

11. The compound of claim 1 wherein the Q is an amidino group B-Q wherein Q is -C(=NH)-NH₂ and B is selected from the group consisting of a substituted or unsubstituted alkyl, alkenyl or alkynyl, a substituted or unsubstituted aryl, arylalkyl, heterocyclic, and heteroaromatic.

12. The compound of claim 11 wherein B is selected from the group consisting of a substituted or unsubstituted alkyl, aryl or arylalkyl.

13. The compound of claim 12 wherein B is a substituted or unsubstituted aryl.

14. The compound of claim 13 wherein B is an aryl.

15. The compound of claim 14 wherein B is selected from the group consisting of phenyl, napthyl, or pyridyl.

16. The compound of claim 15 wherein B is an unsubstituted phenyl.

17. The compound of claim 16 wherein AG-Q is: 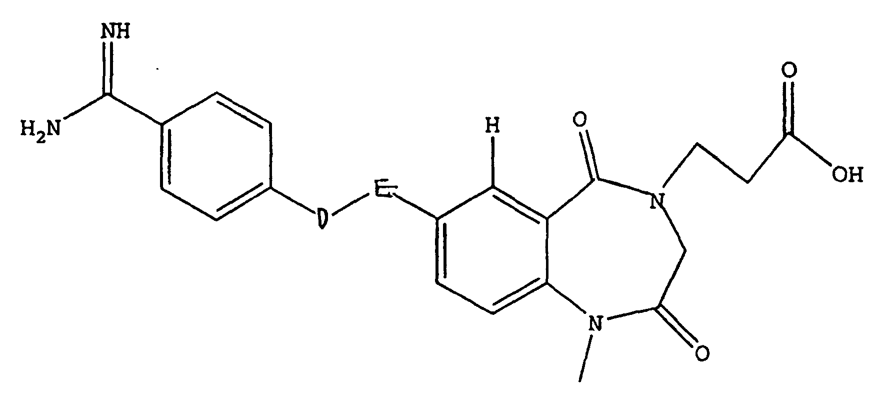 wherein -D-E- is selected from the group consisting of: 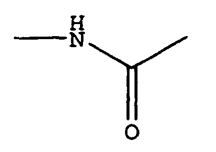 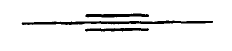 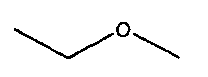 and

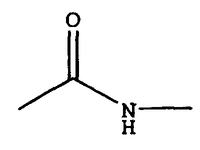

18. The compound of claim 1 wherein AG-Q is a thrombin inhibitor, a platelet aggregation inhibitor, a GPIIb/IIIa receptor blocker, or a Factor Xa inhibitor.

19. A prodrug compound having one of the following two tautomeric structures: 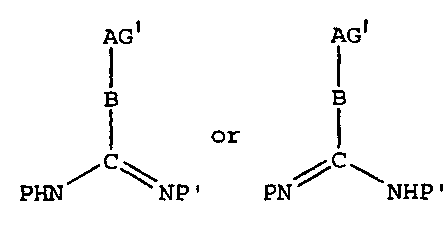 or wherein AG represents a pharmaceutical agent substructure and wherein P and P' are the same or different and are independently selected from H or a trichloroethoxycarbonyl moiety, at least one of P or P' being a trichloroethoxycarbonyl moiety and B is selected from the group consisting of a substituted or unsubstituted alkyl, alkenyl or alkynyl, a substituted or unsubstituted aryl, arylalkyl, heterocyclic, and heteroaromatic.

20. The compound of claim 19 wherein B is selected from the group consisting of a substituted or unsubstituted alkyl, aryl or arylalkyl.

21. The compound of claim 20 wherein B is a substituted or unsubstituted aryl.

22. The compound of claim 21 wherein B is an aryl.

23. The compound of claim 22 wherein B is selected from the group consisting of phenyl, napthyl, or pyridyl.

24. The compound of claim 23 wherein B is an unsubstituted phenyl.

25. A pharmaceutical composition in unit dosage form adapted for oral administration comprising a prodrug of claim 1 and a pharmaceutically acceptable carrier therefor.

26. A compound of any one of claims 1 to 24, for use in a method of medical treatment.

27. A method of preparing a prodrug of a pharmaceutical agent incorporating a basic N-containing functionality selected from the group consisting of an amino, amidino, aminoalkyleneamino, iminoalkyleneamino and guanidino group, comprising reacting said pharmaceutical agent with a compound 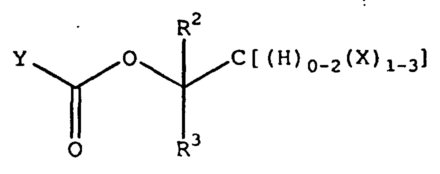 wherein Y is a leaving group; [(H)₀₋₂(X)₁₋₃] is selected from the group consisting of X₃, HX₂ and H₂X; X is a halogen selected from the group consisting of F, Cl, Br, I or a combination thereof; and R² and R³ are the same or different and are selected from the group consisting of H, C₁-C₄ alkyl, C₁-C₄ alkoxy, cyano, halo (F, Cl, Br, I) C₁-C₄ alkyl and aryl.

28. A method of increasing the permeability across lipophilic membranes of a pharmaceutical agent incorporating a basic N-containing functionality selected from the group consisting of an amino, amidino, aminoalkyleneamino, iminoalkyleneamino and guanidino group, comprising reacting said pharmaceutical agent with a compound 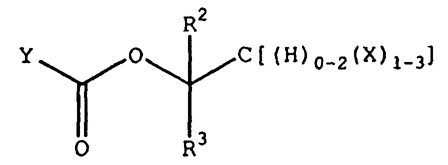 wherein Y is a leaving group; [(H)₀₋₂(X)₁₋₃] is selected from the group consisting of X₃, HX₂ and H₂X; X is a halogen selected from the group consisting of F, Cl, Br, I or a combination thereof; and R² and R³ are the same or different and are selected from the group consisting of H, C₁-C₄ alkyl, C₁-C₄ alkoxy, cyano, halo (F, Cl, Br, I) C₁-C₄ alkyl and aryl.

## Patentansprüche

1. Prodrug-Verbindung der Formel 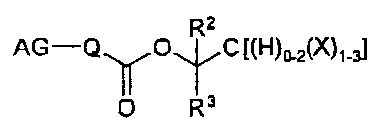 worin [(H)₀₋₂(X)₁₋₃] aus der aus X₃, HX₂ und H₂X bestehenden Gruppe ausgewählt ist und X ein aus der aus F, Cl, Br, I und Kombinationen davon bestehenden Gruppe ausgewähltes Halogen ist; R² und R³ gleich oder unterschiedlich sind und aus der aus H, C₁-C₄-Alkyl, C₁-C₄-Alkoxy, Cyano, halogeniertem (F-, Cl-, Br-, I-) C₁-C₄-Alkyl und Aryl bestehenden Gruppe ausgewählt sind, AG-Q ein pharmazeutischer Wirkstoff ist, worin Q eine aus der aus Amino-, Aminido-, Aminoalkylenamino-, Iminoalkylenamino- und Guanidinogruppen bestehenden Gruppe ausgewählte, basische, N-hältige Funktionalität ist, worin die Prodrug-Gruppierung über ein N-Atom an die N-hältige Funktionalität gebunden ist.

2. Verbindung nach Anspruch 1, worin X Cl ist und R² und R³ gleich oder unterschiedlich sind und aus der aus H, C₁-C₄-Alkyl und Aryl bestehenden Gruppe ausgewählt sind.

3. Verbindung nach Anspruch 2, worin das C₁-C₄-Alkyl aus der aus Methyl, Ethyl, Propyl und Butyl bestehenden Gruppe ausgewählt ist.

4. Verbindung nach Anspruch 3, worin das C₁-C₄-Alkyl Methyl ist.

5. Verbindung nach Anspruch 4, worin R² und R³ gleich sind.

6. Verbindung nach Anspruch 5, worin R² und R³ H sind.

7. Verbindung nach Anspruch 6, worin [(H)₀₋₂(X)₁₋₃] X₃ ist.

8. Verbindung nach Anspruch 1, worin Y aus der aus Amidino- und Guanidinogruppe bestehenden Gruppe ausgewählt ist.

9. Verbindung nach Anspruch 8, worin Q eine Amidinogruppe zur Bindung der Prodrug-Gruppierung ist und aus folgender Gruppe ausgewählt ist:

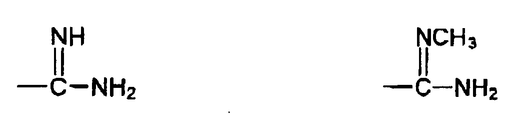

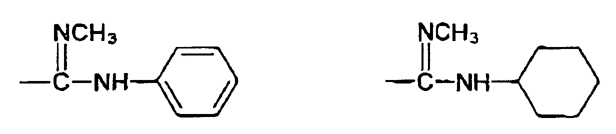

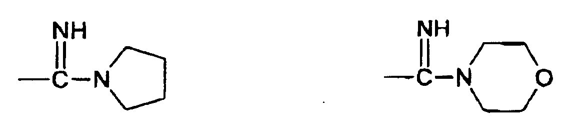

10. Verbindung nach Anspruch 8, worin Y aus folgender Gruppe ausgewählt ist:

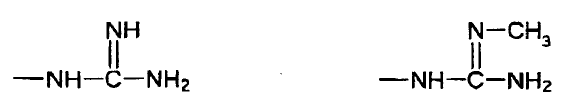

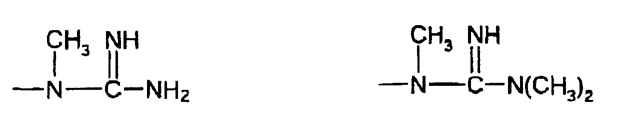

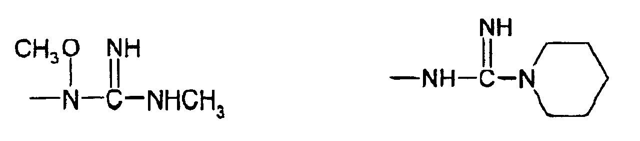

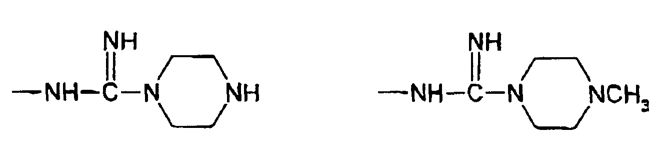

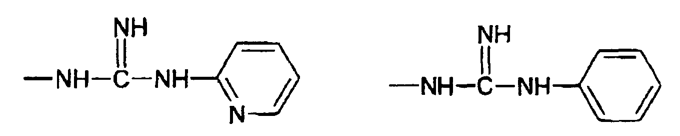

11. Verbindung nach Anspruch 1, worin Q eine Amidinogruppe B-Q ist, worin Q -C(=NH)-NH₂ ist und B aus der aus substituiertem oder unsubstituiertem Alkyl, Alkenyl und Alkinyl, substituierten oder unsubstituierten Arylen, Arylalkylen, Heterozyklen und Heteroaromaten bestehenden Gruppe ausgewählt ist.

12. Verbindung nach Anspruch 11, worin B aus der aus substituiertem oder unsubstituiertem Alkyl, Aryl und Arylkalkyl bestehenden Gruppe ausgewählt ist.

13. Verbindung nach Anspruch 12, worin B substituiertes oder unsubstituiertes Aryl ist.

14. Verbindung nach Anspruch 13, worin B Aryl ist.

15. Verbindung nach Anspruch 14, worin B aus der aus Phenyl, Naphthyl und Pyridyl bestehenden Gruppe ausgewählt ist.

16. Verbindung nach Anspruch 15, worin B unsubstituiertes Phenyl ist.

17. Verbindung nach Anspruch 16, worin AG-Q Folgendes ist: 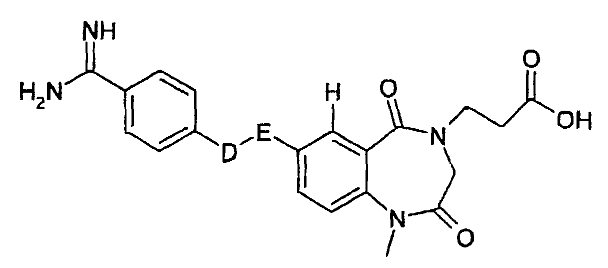 worin D-E aus folgender Gruppe ausgewählt ist: 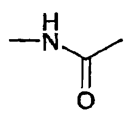 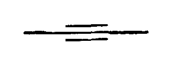 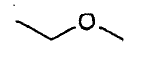 und

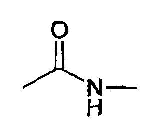

18. Verbindung nach Anspruch 1, worin AG-Q ein Thrombininhibitor, ein Btutplättchenaggregationshemmer, ein GPIIb/IIIa-Rezeptor-Blocker oder ein Faktor-Xa-Inhibitor ist.

19. Prodrug-Verbindung mit einer der folgenden beiden tautomeren Strukturen: 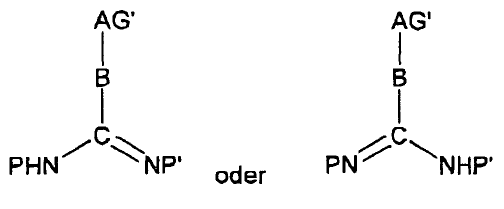 oder worin AG für eine Substruktur eines pharmazeutischen Wirkstoffs steht und worin P und P' gleich oder unterschiedlich sind und unabhängig voneinander aus H und der Trichlorethoxycarbonyl-Gruppierung ausgewählt sind, zumindest eines von P und P' die Trichlorethoxycarbonyl-Gruppierung ist und B aus der aus substituiertem oder unsubstituiertem Alkyl, Alkenyl und Alkinyl, substituierten oder unsubstituierten Arylen, Arylalkylen, Heterozyklen und Heteroaromaten bestehenden Gruppe ausgewählt ist.

20. Verbindung nach Anspruch 19, worin B aus der aus substituiertem oder unsubstituiertem Alkyl, Aryl und Arylalkyl bestehenden Gruppe ausgewählt ist.

21. Verbindung nach Anspruch 20, worin B substituiertes oder unsubstituiertes Aryl ist.

22. Verbindung nach Anspruch 21, worin B Aryl ist.

23. Verbindung nach Anspruch 22, worin B aus der aus Phenyl, Naphthyl und Pyridyl bestehenden Gruppe ausgewählt ist.

24. Verbindung nach Anspruch 23, worin B unsubstituiertes Phenyl ist.

25. Pharmazeutische Zusammensetzung in zur oralen Verabreichung geeigneter Einzeldosisform, umfassend ein Prodrug nach Anspruch 1 und einen pharmazeutisch annehmbaren Träger dafür.

26. Verbindung nach einem der Ansprüche 1 bis 24 zur Verwendung in einem medizinischen Behandlungsverfahren.

27. Verfahren zur Herstellung eines Prodrugs aus einem pharmazeutischen Wirkstoff, das eine aus der aus Amino-, Aminido-, Aminoalkylenamino-, Iminoalkylenamino- und Guanidinogruppen bestehenden Gruppe ausgewählte, basische, N-hältige Funktionalität enthält, umfassend das Umsetzen des pharmazeutischen Wirkstoffs mit einer Verbindung 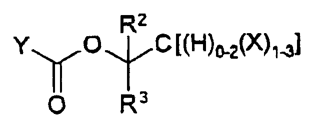 worin Y eine Abgangsgruppe ist; [(H)₀₋₂(X)₁₋₃] aus der aus X₃, HX₂ und H₂X bestehenden Gruppe ausgewählt ist; X ein aus der aus F, Cl, Br, 1 und Kombinationen davon bestehenden Gruppe ausgewähltes Halogen ist; und R² und R³ gleich oder unterschiedlich sind und aus der aus H, C₁-C₄-Alkyl, C₁-C₄-Akoxy, Cyano, halogeniertem (F-, Cl-, Br-, I-) C₁-C₄-Alkyl und Aryl bestehenden Gruppe ausgewählt sind.

28. Verfahren zur Erhöhung der Durchlässigkeit von lipophilen Membranen für einen pharmazeutischen Wirkstoff, der eine aus der aus Amino-, Aminido-, Aminoalkylenamino-, Iminoalkylenamino- und Guanidinogruppen bestehenden Gruppe ausgewählte, basische, N-hältige Funktionalität enthält, umfassend das Umsetzen des pharmazeutischen Wirkstoffs mit einer Verbindung 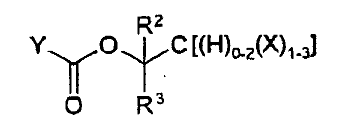 worin Y eine Abgangsgruppe ist; [(H)₀₋₂(X)₁₋₃] aus der aus X₃, HX₂ und H₂X bestehenden Gruppe ausgewählt ist; X ein aus der aus F, Cl, Br, I und Kombinationen davon bestehenden Gruppe ausgewähltes Halogen ist; und R² und R³ gleich oder unterschiedlich sind und aus der aus H, C₁-C₄-Alkyl, C₁-C₄-Akoxy, Cyano, halogeniertem (F-, Cl-, Br-, I-) C₁-C₄-Alkyl und Aryl bestehenden Gruppe ausgewählt sind.

## Revendications

1. Composé précurseur médicamenteux de formule : 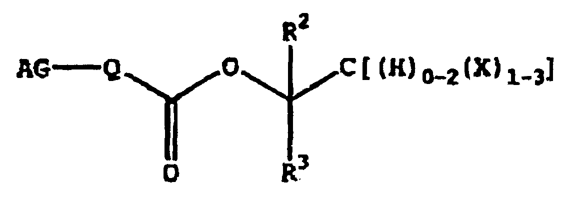 dans laquelle [(H)₀₋₂(X)₂₋₃] est choisi dans le groupe consistant en X₃, HX₂ et H₂X, et X représente un halogène choisi dans le groupe consistant en F, Cl, Br, I ou une de leurs associations ; R² et R³ sont identiques ou différents et sont choisis dans le groupe consistant en H, des groupes alkyle en C₁ à C₄, alkoxy en C₁ à C₄, cyano, halogéno(F, Cl, Br, I)-alkyle en C₁ à C₄ et aryle, AG-Q représente un agent pharmaceutique dans lequel Q représente une fonctionnalité basique contenant N, choisie dans le groupe consistant en des groupes amino, amidino, aminoalkylèneamino, iminoalkylèneamino et guanidino, le groupement précurseur médicamenteux étant lié à la fonctionnalité contenant N par un atome de N.

2. Composé suivant la revendication 1, dans lequel X représente Cl, et R² et R³ sont identiques ou différents et sont choisis dans le groupe consistant en H, des groupes alkyle en C₁ à C₄ et aryle.

3. Composé suivant la revendication 2, dans lequel le groupe alkyle en C₁ à C₄ est choisi dans le groupe consistant en les groupes méthyle, éthyle, propyle et butyle.

4. Composé suivant la revendication 3, dans lequel le groupe alkyle en C₁ à C₄ est un groupe méthyle.

5. Composé suivant la revendication 4, dans lequel R² et R³ sont identiques.

6. Composé suivant la revendication 5, dans lequel R² et R³ représentent H.

7. Composé suivant la revendication 6, dans lequel [(H)₀₋₂(X)₁₋₃] représente X₃.

8. Composé suivant la revendication 1, dans lequel Q est choisi dans le groupe consistant en un groupe amidino et un groupe guanidino.

9. Composé suivant la revendication 8, dans lequel Q représente un groupe amidino pour la liaison au groupement précurseur médicamenteux et est choisi dans le groupe consistant en :

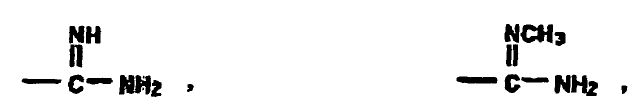

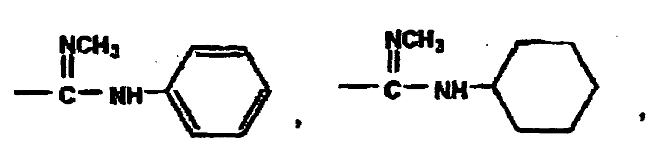

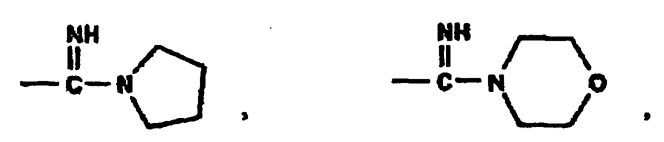

10. Composé suivant la revendication 8, dans lequel Q est choisi dans le groupe consistant en :

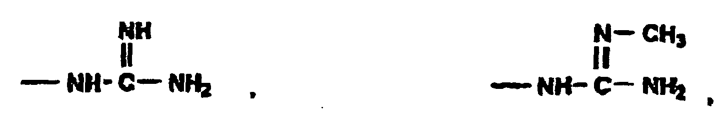

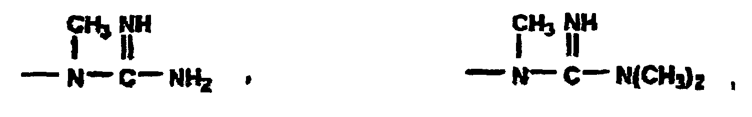

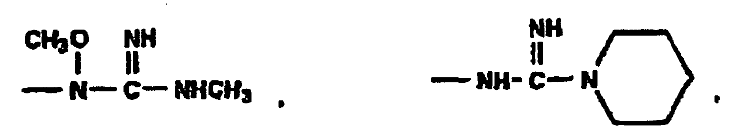

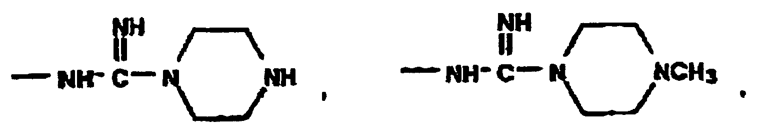

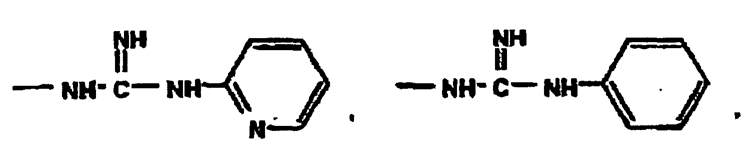

11. Composé suivant la revendication 1, dans lequel le groupe Q est un groupe amidino B-Q dans lequel Q représente un groupe -C(=NH)-NH₂ et B est choisi dans le groupe consistant en un groupe alkyle, alcényle ou alcynyle substitué ou non substitué, et un groupe aryle, arylalkyle, hétérocyclique ou hétéroaromatique substitué ou non substitué.

12. Composé suivant la revendication 11, dans lequel B est choisi dans le groupe consistant en des groupes alkyle, aryle ou arylalkyle substitués ou non substitués.

13. Composé suivant la revendication 12, dans lequel B représente un groupe aryle substitué ou non substitué.

14. Composé suivant la revendication 13, dans lequel B représente un groupe aryle.

15. Composé suivant la revendication 14, dans lequel B est choisi dans le groupe consistant en des groupes phényle, naphtyle et pyridyle.

16. Composé suivant la revendication 15, dans lequel B représente un groupe phényle non substitué.

17. Composé suivant la revendication 16, dans lequel AG-Q représente un groupe : 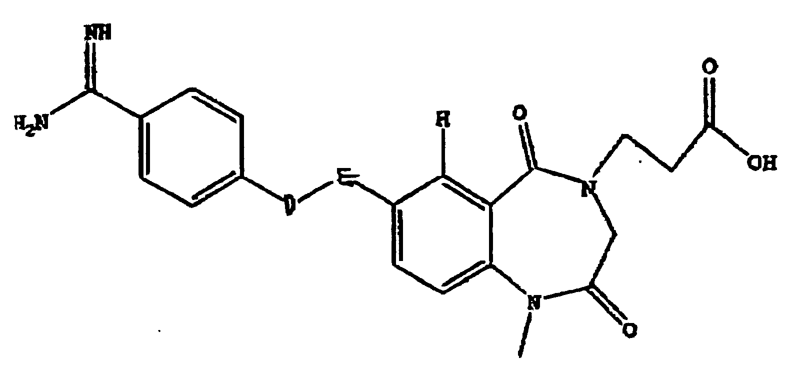 dans lequel -D-E- est choisi dans le groupe consistant en : 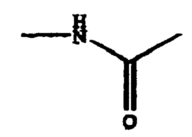 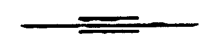 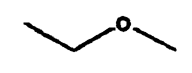 et

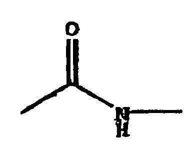

18. Composé suivant la revendication 1, dans lequel AG-Q est un inhibiteur de thrombine, un inhibiteur d'agrégation plaquettaire, un agent de blocage des récepteurs des GPIIb/IIIa ou un inhibiteur du facteur Xa.

19. Composé précurseur médicamenteux ayant une des deux structures tautomères suivantes : 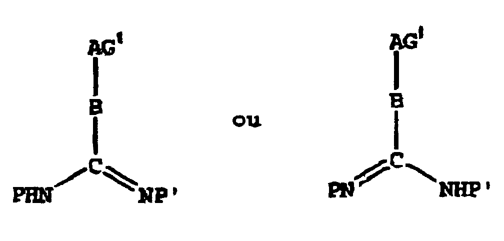 dans lesquelles AG représente une sous-structure d'agent précurseur médicamenteux, et P et P' sont identiques ou différents et sont choisis indépendamment entre H et un groupement trichloréthoxycarbonyle, au moins un de P et P' étant un groupement trichloréthoxycarbonyle et B étant choisi dans le groupe consistant en un groupe alkyle, alcényle ou alcynyle substitué ou non substitué, un groupe aryle substitué ou non substitué, un groupe arylalkyle, hétérocyclique et hétéroaromatique.

20. Composé suivant la revendication 19, dans lequel B est choisi dans le groupe Consistant en des groupes alkyle, aryle ou arylalkyle substitués ou non substitués.

21. Composé suivant la revendication 20, dans lequel B représente un groupe aryle substitué ou non substitué.

22. Composé suivant la revendication 21, dans lequel B représente un groupe aryle.

23. Composé suivant la revendication 22, dans lequel B est choisi dans le groupe consistant en les groupes phényle, naphtyle et pyridyle.

24. Composé suivant la revendication 23, dans lequel B représente un groupe phényle non substitué.

25. Composition pharmaceutique sous une forme posologique unitaire adaptée à l'administration orale, comprenant un précurseur médicamenteux de la revendication 1 et un support pharmaceutiquement acceptable à cette fin.

26. Composé suivant l'une quelconque des revendications 1 à 24, destiné à être utilisé dans une méthode de traitement médical.

27. Procédé pour la préparation d'un précurseur médicamenteux d'un agent pharmaceutique renfermant une fonctionnalité basique contenant N choisie dans le groupe consistant en des groupes amino, amidino, aminoalkylèneamino, iminoalkylèneamino et guanidino, comprenant la réaction dudit agent pharmaceutique avec un composé de formule 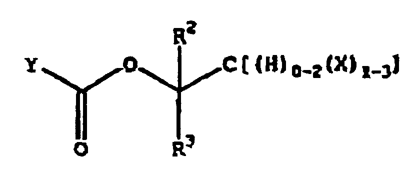 dans laquelle Y représente un groupe partant ; [(H)₀₋₂(X)₁₋₃] est choisi dans le groupe consistant en X₃, HX₂ et H₂X ; X représente un halogène choisi dans le groupe consistant en F, Cl, Br, I ou une de leurs associations ; et R² et R³ sont identiques ou différents et sont choisis dans le groupe consistant en H, des groupes alkyle en C₁ à C₄, alkoxy en C₁ à C₄, cyano, halogéno(F, Cl, Br, I)-alkyle en C₁ à C₄ et aryle.

28. Méthode pour accroître la perméabilité à travers des membranes lipophiles d'un agent pharmaceutique renfermant une fonctionnalité basique contenant N choisie dans le groupe consistant en des groupes amino, amidino, aminoalkylèneamino, iminoalkylèneamino et guanidino, comprenant la réaction dudit agent pharmaceutique avec un composé de formule 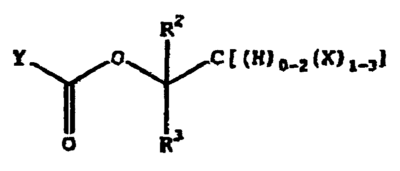 dans laquelle Y représente un groupe partant ; [(H)₀₋₂(x)₁₋₃] est choisi dans le groupe consistant en X₃, HX₂ et H₂X ; X représente un halogène choisi dans le groupe consistant en F, Cl, Br, I ou une de leurs associations ; et R² et R³ sont identiques ou différents et sont choisis dans le groupe consistant en H, des groupes alkyle en C₁ à C₄, alkoxy en C₁ à C₄, cyano, halogéno(F, Cl, Br, I)-alkyle en C₁ à C₄ et aryle.
